# EUROPEAN PATENT SPECIFICATION

(11) **EP 4 072 673 B1**
(45) Date of publication and mention of the grant of the patent: **04.11.2026**
(21) Application number: 20897805.6
(22) Date of filing: 09.12.2020
(51) Int. Cl.: G01N 33/575, A61P 7/00, A61P 7/02, A61P 35/00, C12Q 1/6883, C12Q 1/6886, G01N 33/86, G01N 33/57525

(54) **IDENTIFYING AND MODULATING THROMBOTIC CONDITIONS IN A CANCER PATIENT**
IDENTIFIZIERUNG UND MODULATION VON THROMBOSEN BEI EINEM KREBSPATIENT
IDENTIFICATION ET MODULATION D'ÉTATS THROMBOTIQUES CHEZ UN PATIENT ATTEINT D'UN CANCER

(30) Priority: 09.12.2019 US 201962945482 P
(43) Date of publication of application: 19.10.2022
(73) Proprietor: Beth Israel Deaconess Medical Center, Inc., Boston, MA 02215 (US)
(72) Inventor: ZWICKER, Jeffrey I., Boston, Massachusetts 02215 (US); FLAUMENHAFT, Robert, Boston, Massachusetts 02215 (US)
(74) Representative: HGF
(86) International application number: PCT/US2020/064101
(87) International publication number: WO 2021/119189

(56) References cited:
- WO-A1-2017/027379
- WO-A2-2012/174282
- US-A1- 2014 350 129
- US-A1- 2016 041 153
- US-A1- 2019 241 633
- BAATEN, CONSTANCE C. F. M. J., STUART MEACHAM, SUSANNE M. DE WITT,, MARION A. H. FEIJGE, DAVID J. ADAMS, JAN-WILLEM N. AKKERMAN, J: "A synthesis approach of mouse studies to identify genes and proteins in arterial thrombosis and bleeding", BLOOD, vol. 132, no. 24, 1 October 2018 (2018-10-01), pages 35 - 46, XP055836634
- RAZAK ET AL.: "Cancer-Associated Thrombosis: An Overview of Mechanisms, Risk Factors, and Treatment", CANCERS, vol. 10, no. 380, 11 October 2018 (2018-10-11), pages 1 - 21, XP055836639

## Description

### CROSS REFERENCE TO RELATED APPLICATIONS

This application claims the benefit under 35 U.S.C. §119(e) of U.S. Provisional Patent Application No. 62/945,482, filed December 9, 2019.

### FIELD OF THE DISCLOSURE

The present disclosure relates to methods for determining risk of a thrombotic event in a cancer patient, and an effective amount of isoquercetin for treatment of a cancer patient identified as being at risk of a thrombotic event using a method of the invention.

### GOVERNMENT SUPPORT CLAUSE

This invention was made with government support under Grant Nos. HL112302, and HL143365 awarded by the NIH. The Government has certain rights in this invention.

### BACKGROUND

Thrombosis is a significant cause of mortality in cancer patients. Bick, N Engl J Med 349:109-111 (2003). For example, serious, life-threatening thrombotic events occur in approximately 6% of lung cancer patients. Alguire et al., J Clin Oncol 2004 Vol 22 (July 15th Supplement) No. 14S: 8082. Cancer patients often exhibit hypercoagulation, in which the coagulation system has an increased clotting tendency. Rickles and Edwards, Blood 62:14-31 (1983). Markers of hypercoagulation correlate with poor patient outcome for at least some cancers. Bick, Semin Thromb Hemostat 18:353-372 (1992); Buccheri et al., Cancer 97:3044-3052 (2003); Wojtukiewicz, Blood Coagul Fibrinolysis 3:429-437 (1992). Causes of hypercoagulation include the cancer itself and the cancer treatments (e.g., chemotherapy). Hypercoagulation results in an increased risk of thrombotic events, which can be further exacerbated when patients become bed-ridden. When not contraindicated, anticoagulant therapy has conferred survival benefit in some cancers. Lebeau et al., Cancer 74:38-45 (1994); Chahinian et al., J Clin Oncol 7:993-1002 (1989). However, therapeutic options are often limited because many cancer patients are at an elevated risk of major bleeding, precluding administration of anticoagulants that could otherwise be given prophylactically to reduce the risk of thrombosis. Thus, presently available methods for diagnosis and prevention of thrombosis in cancer patients are unsatisfactory, and thus there is a need for new diagnostics and therapies. Such diagnostics and therapies would enhance cancer patient survival and promote better quality of life. WO 2017/027379 discloses a method for screening for cancer-associated thrombosis using the biomarker H3Cit.

### BRIEF DESCRIPTION OF THE DRAWINGS

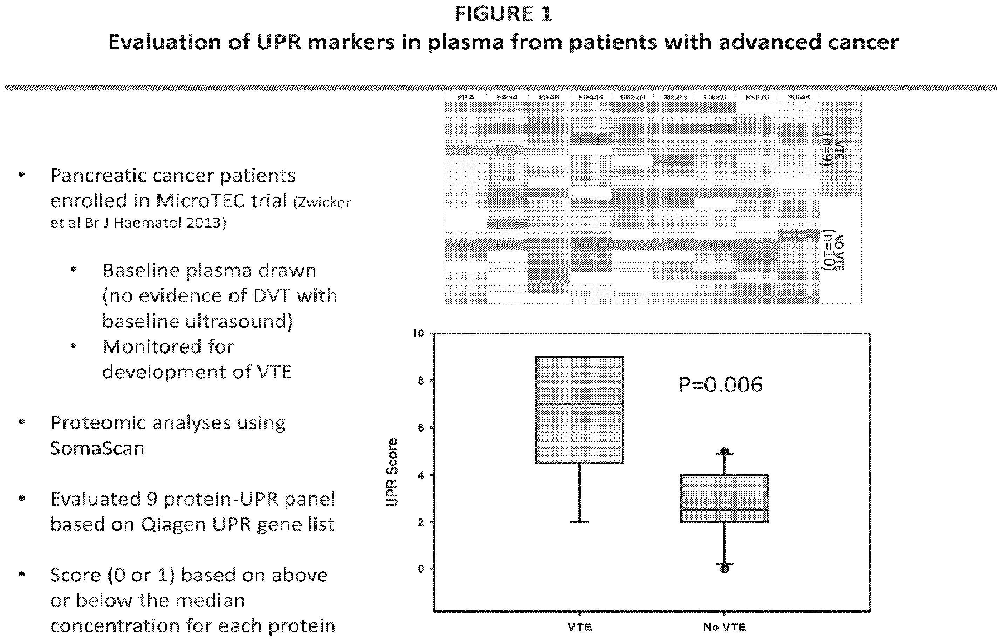

Figure **1** shows a heat map and UPR panel evaluating the UPR markers in plasma from patients with advanced cancer.

### SUMMARY

An embodiment provided herein is a method of determining risk for a thrombotic event in a cancer patient comprising: detecting in a serum or plasma sample obtained from a patient with cancer elevated levels of PPIA, PDIA3 and at least one of EIF5A, EIF4H, EIF4a3, UBE2N, UBE2L3, UBE2I, and HSP70 when compared with a baseline, reference, or control levels of PPIA, PDIA3 and at least one of EIF5A, EIF4H, EIF4a3, UBE2N, UBE2L3, UBE2I, and HSP70; and diagnosing the patient as at risk of a thrombotic event when PPIA, PDIA3 and at least one of EIF5A, EIF4H, EIF4a3, UBE2N, UBE2L3, UBE2I, and HSP70 are elevated over a baseline, control, or reference level.

An additional embodiment provided herein is an effective amount of isoquercetin and optionally an antithrombotic agent for use in a method of treating a thrombotic condition in a cancer patient, wherein the cancer patient has been diagnosed as being at risk of a thrombotic event by a method comprising detecting in a serum or plasma sample of a patient with cancer elevated levels of PPIA, PDIA3 and at least one of EIF5A, EIF4H, EIF4a3, UBE2N, UBE2L3, UBE2I, and HSP70 when compared with a baseline, reference, or control levels of PPIA, PDIA3 and at least one of EIF5A, EIF4H, EIF4a3, UBE2N, UBE2L3, UBE2I, and HSP70; and diagnosing the patient as at-risk of a thrombotic condition when PPIA, PDIA3 and at least one of EIF5A, EIF4H, EIF4a3, UBE2N, UBE2L3, UBE2I, and HSP70 are elevated over a baseline, control or reference level.

A further embodiment provided herein is a method for determining risk of a thrombotic condition in a cancer patient undergoing treatment patient with an effective amount of isoquercetin and optionally an antithrombotic agent, wherein the method comprises detecting in a serum or plasma sample of the patient with cancer elevated levels of PPIA, PDIA3 and at least one of EIF5A, EIF4H, EIF4a3, UBE2N, UBE2L3, UBE2I, and HSP70 when compared with a baseline, reference, or control levels of PPIA, PDIA3 and at least one of EIF5A, EIF4H, EIF4a3, UBE2N, UBE2L3, UBE2I, and HSP70; and diagnosing the patient as at-risk of a thrombotic condition when PPIA, PDIA3 and at least one of EIF5A, EIF4H, EIF4a3, UBE2N, UBE2L3, UBE2I, and HSP70 are elevated over a baseline, control or reference level; wherein the method of determining risk is repeated weekly, bi-weekly, monthly, or as long as indicated throughout the course of treatment.

In certain embodiments, the patient exhibits no severe adverse events (grade 3 or 4 toxicities) during treatment.

In certain embodiments, the patient exhibits no primary venous thromboembolism (VTE) during treatment.

In certain embodiments, the patient exhibits no major hemorrhages during treatment.

In an additional embodiment, the method of the present invention comprises contacting the serum or plasma sample with (a) a solid support coated with polyclonal or monoclonal antibodies, wherein the antibodies comprise antibodies specific to PPIA, PDIA3 and at least one of EIF5A, EIF4H, EIF4a3, UBE2N, UBE2L3, UBE2I, and HSP70 (b) polyclonal or monoclonal antibody-substrate conjugates, wherein the substrate comprises a chromogenic or fluorescent reagent, and wherein the conjugates are reactive with the antibodies of (a); and (c) PPIA, PDIA3 and at least one of EIF5A, EIF4H, EIF4a3, UBE2N, UBE2L3, UBE2I, and HSP70 as antigen standards. In an embodiment, wherein the solid support comprises a microtiter plate, wherein the conjugates comprise alkaline phosphatase, wherein the chromogenic reagent comprises p-nitrophenyl-phosphate; and ii) assaying the reaction of the conjugates with the sample.

In certain embodiments, the antibodies of (a) further comprise antibodies specific to soluble P selectin.

In certain embodiments, the solid support is a microtiter plate or membrane. In certain embodiments, the solid support is a bead or particle. In certain embodiments, the assay is an ELISA. In certain embodiments, the solid support is a microbead array.

In certain embodiments, the method further comprises contacting the serum or plasma sample with the polyclonal or monoclonal antibody-substrate conjugates, and assaying the reaction of the conjugates with the sample.

In certain embodiments, the method further comprises contacting the antigen standards with the solid support and the conjugates, and assaying the relative levels of PPIA, PDIA3 and at least one of EIF5A, EIF4H, EIF4a3, UBE2N, UBE2L3, UBE2I, and HSP70 in the sample relative to the antigen standards.

### DETAILED DESCRIPTION

Before the present invention is described, it is to be understood that this invention is not limited to the particular processes, formulations, compositions, or methodologies described, as these may vary. It is also to be understood that the terminology used in the description is for the purpose of describing the particular versions or embodiments only, and is not intended to limit the scope of embodiments herein which will be limited only by the appended claims. Unless defined otherwise, all technical and scientific terms used herein have the same meanings as commonly understood by one of ordinary skill in the art. Although any methods and materials similar or equivalent to those described herein can be used in the practice or testing of embodiments of embodiments herein, the preferred methods, devices, and materials are now described. Nothing herein is to be construed as an admission that embodiments herein is not entitled to antedate such disclosure by virtue of prior invention.

It must also be noted that as used herein and in the appended claims, the singular forms "a," "an," and "the" include plural reference unless the context clearly dictates otherwise.

As used herein, the term "about" means plus or minus 10% of the numerical value of the number with which it is being used. Therefore, about 50% means in the range of 45%-55%.

The term "subject" as used herein includes, but is not limited to, humans (also typically referred to as "patients") and non-human vertebrates such as wild, domestic, and farm animals. In certain embodiments, the subject described herein is an animal. In certain embodiments, the subject is a mammal. In certain embodiments, the subject is a human. In certain embodiments, the subject is a non-human animal. In certain embodiments, the subject is a non-human mammal. In certain embodiments, the subject is a domesticated animal, such as a dog, cat, cow, pig, horse, sheep, or goat. In certain embodiments, the subject is a companion animal such as a dog or cat. In certain embodiments, the subject is a livestock animal such as a cow, pig, horse, sheep, or goat. In certain embodiments, the subject is a zoo animal. In another embodiment, the subject is a research animal such as a rodent, dog, or non-human primate. In certain embodiments, the subject is a non-human transgenic animal such as a transgenic mouse or transgenic pig.

The terms "treat," "treated," or "treating" as used herein refers to both therapeutic treatment and prophylactic or preventative measures, wherein the object is to inhibit, prevent or slow down or reduce the full effect or likelihood of (lessen) any undesired physiological condition, disorder or disease, or to improve, inhibit, or otherwise obtain beneficial or desired clinical results. For the purposes of this invention, beneficial or desired clinical results include, but are not limited to, improvement or alleviation of symptoms; diminishment of the extent of the condition, disorder or disease; stabilization (i.e., not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment.

The terms "screen" and "screening" and the like as used herein means to test a subject or patient to determine if they have a particular illness or disease, or a particular manifestation of an illness or disease. The term also means to test an agent to determine if it has a particular action or efficacy.

The terms "identification", "identify", "identifying" and the like as used herein means to recognize a disease state or a clinical manifestation or severity of a disease state in a subject or patient. The term also is used in relation to test agents and their ability to have a particular action or efficacy.

The terms "prediction", "predict", "predicting" and the like as used herein means to tell in advance based upon special knowledge.

The term "reference value" or "control value" as used herein means an amount or a quantity of a particular protein or nucleic acid in a sample from a healthy control or healthy donor, or in certain instances from an advanced cancer patient who does not exhibit VTE or other thrombotic condition, for a period of time.

The terms "healthy control", is a human subject who is not suffering from cancer or any other cancer-related condition.

As used herein, the term "isolated" and the like means that the referenced material is free of components found in the natural environment in which the material is normally found. In particular, isolated biological material is free of cellular components. In the case of nucleic acid molecules, an isolated nucleic acid includes a PCR product, an isolated mRNA, a cDNA, an isolated genomic DNA, or a restriction fragment. In another embodiment, an isolated nucleic acid is preferably excised from the chromosome in which it may be found. Isolated nucleic acid molecules can be inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated material may be, but need not be, purified.

The term "purified" and the like as used herein refers to material that has been isolated under conditions that reduce or eliminate unrelated materials, i.e., contaminants. For example, a purified protein is preferably substantially free of other proteins or nucleic acids with which it is associated in a cell; a purified nucleic acid molecule is preferably substantially free of proteins or other unrelated nucleic acid molecules with which it can be found within a cell. As used herein, the term "substantially free" is used operationally, in the context of analytical testing of the material. Preferably, purified material substantially free of contaminants is at least 50% pure; more preferably, at least 90% pure, and more preferably still at least 99% pure. Purity can be evaluated by chromatography, gel electrophoresis, immunoassay, composition analysis, biological assay, and other methods known in the art.

The terms "expression profile" or "gene expression profile" refers to any description or measurement of one or more of the genes that are expressed by a cell, tissue, or organism under or in response to a particular condition. Expression profiles can identify genes that are up-regulated, down-regulated, or unaffected under particular conditions. Gene expression can be detected at the nucleic acid level or at the protein level. The expression profiling at the nucleic acid level can be accomplished using any available technology to measure gene transcript levels. For example, the method could employ *in situ* hybridization, Northern hybridization or hybridization to a nucleic acid microarray, such as an oligonucleotide microarray, or a cDNA microarray. Alternatively, the method could employ reverse transcriptase-polymerase chain reaction (RT-PCR) such as fluorescent dye-based quantitative real time PCR (TaqMan^{®} PCR). In the Examples section provided below, nucleic acid expression profiles were obtained using Affymetrix GeneChip^{®} oligonucleotide microarrays. The expression profiling at the protein level can be accomplished using any available technology to measure protein levels, e.g., using peptide-specific capture agent arrays.

The terms "gene signature" and "signature genes" will be used interchangeably herein and mean the particular transcripts that have been found to be differentially expressed in some cancer patients.

### UPR biomarkers

Nine UPR biomarkers (human) that have been found to be elevated in plasma samples of advanced cancer patients are: PPIA, EIF5A, EIF4H, EIF4a3, UBE2N, UBE2L3, UBE2I, HSP70, and PDIA3. These biomarkers were found to be elevated in plasma samples from patients with advanced cancer that later exhibited VTE, as compared to non-elevated levels of the same UPR biomarker proteins in plasma samples from patients with advanced cancer that did not exhibit VTE (e.g., VTE was monitored for 2 months, but in some embodiments, it would be desirable to keep testing every 2 weeks, or monthly throughout the duration of treatment), and as such serve as the baseline reference samples. The elevated protein UPR levels are referred to herein as the UPR biomarker panel, which is predictive for developing a thrombotic condition, such as VTE. tThe UPR biomarker panel comprises any combination of PPIA, PDIA3 and at least one of EIFSA, EIF4H, EIF4a3, UBE2N, UBE2L3, UBE2I, and HSP70. In some embodiments, the UPR biomarker panel comprises PPIA, EIF5A, EIF4H, EIF4a3, UBE2N, UBE2L3, UBE2I, HSP70, and PDIA3. In some embodiments, the UPR biomarker panel comprises PPIA, EIF4H, PDIA3 and at least one of EIF5A, EIF4a3, UBE2N, UBE2L3, UBE2I, and HSP70.

The amino acid sequence for PPIA can be found at: P62937; and the Gene ID is 5478. The amino acid sequence for EIF5A can be found at: P63241; and the Gene ID is 1984.The amino acid sequence for EIF4H can be found at: Q15056 and the Gene ID is 7458. The amino acid sequence for EIF4a3 can be found at: P38919; and the Gene ID is 9775. The amino acid sequence for UBE2N can be found at: P61088; and the Gene ID is 7334. The amino acid sequence for UBE2L3 can be found at: P68036; and the Gene ID is 7332. The amino acid sequence for UBE2I can be found at: P63279; and the Gene ID is 7329. The amino acid sequence for HSP70 can be found at: P0DMV8/9; and the Gene ID is 3303. The amino acid sequence for PDIA3 can be found at: P30101; and the Gene ID is 2923.

The terms "gene", "gene transcript", and "transcript" are used somewhat interchangeable in the application. The term "gene", also called a "structural gene" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. "Transcript" or "gene transcript" is a sequence of RNA produced by transcription of a particular gene. Thus, the expression of the gene can be measured via the transcript.

The term "antisense DNA" is the non-coding strand complementary to the coding strand in double-stranded DNA.

The term "genomic DNA" as used herein means all DNA from a subject including coding and non-coding DNA, and DNA contained in introns and exons.

The term "nucleic acid hybridization" refers to anti-parallel hydrogen bonding between two single-stranded nucleic acids, in which A pairs with T (or U if an RNA nucleic acid) and C pairs with G. Nucleic acid molecules are "hybridizable" to each other when at least one strand of one nucleic acid molecule can form hydrogen bonds with the complementary bases of another nucleic acid molecule under defined stringency conditions. Stringency of hybridization is determined, e.g., by (i) the temperature at which hybridization and/or washing is performed, and (ii) the ionic strength and (iii) concentration of denaturants such as formamide of the hybridization and washing solutions, as well as other parameters. Hybridization requires that the two strands contain substantially complementary sequences. Depending on the stringency of hybridization, however, some degree of mismatches may be tolerated. Under "low stringency" conditions, a greater percentage of mismatches are tolerable (*i.e.,* will not prevent formation of an anti-parallel hybrid).

The term "inhibit" includes the administration of a compound according to embodiments described herein to prevent the onset of the symptoms, alleviating the symptoms, or eliminating the disease, condition or disorder.

By "pharmaceutically acceptable", it is meant the carrier, diluent or excipient must be compatible with the other ingredients of the topical formulation and not deleterious to the recipient thereof.

The term "blood thinning medication" refers to an antiplatelet drug, e.g., clopidogrel bisulfate, heparin, warfarin, enoxaparin, abciximab, eptifibatide, tirofiban, prasugrel, ticlopidine, beraprost, prostacyclin, iloprost, treprostinil, aspirin, aloxiprin, carbasalate calcium, indobufen, triflusal, dipyridamole, picotamide, terutroban, cilostazol, cloricromen, ditazole; or an anticoagulant, e.g., acenocoumarol, coumatetralyl, dicoumarol, ethyl biscoumacetate, phenprocoumon, clorindione, diphenadione, phenindione, tioclomarol, bemiparin, certoparin, dalteparin, nadroparin, parnaparin, reviparin, tinzaparin, fondaparinux, idraparinux, danaparoid, sulodexide, dermatan sulfate, apixaban, betrixaban, edoxaban, otamixaban, rivaroxaban, bivalirudin, lepirudin, desirudin, argatroban, dabigatran, melagatran, ximelagatran, regimen 1 (REG1; a combination of RB-006, a Factor IXa antagonist, and its oligonucleotide active control agent RB-007), defibrotide, ramatroban, antithrombin III, a Factor V inhibitor, a Factor IXa inhibitor, a Factor X inhibitor, a Factor XI inhibitor, a Factor XIII inhibitor, or drotrecogin alfa.

The term "thrombotic disorder" refers to many distinct conditions that cause or increase the risk of a venous or arterial thrombotic event, including but not limited to, atrial fibrillation, thrombosis due to a mechanical heart valve, myocardial infarction, unstable angina, deep vein thrombosis, acute ischemic stroke, pulmonary embolism, atherosclerosis, factor V Leiden, antithrombin III deficiency, protein C deficiency, protein S deficiency, prothrombin gene mutation (G20210A), hyperhomocysteinemia, antiphospholipid antibody syndrome, anticardiolipin antibody, thrombosis syndrome, lupus anticoagulant syndrome, malignancy, major surgery, immobilization, oral contraceptive use, thalidomide use, especially in combination with dexamethasone, heparin-induced thrombocytopenia, pregnancy, myeloproliferative disorders, inflammatory bowel disease, nephrotic syndrome, paroxysmal nocturnal hemoglobinuria, hyperviscosity syndrome, Waldenstrom's macroglobulinemia, and trauma. The term "thrombotic disorder" also refers to thrombosis induced by cancer, e.g., multiple myeloma and other hematologic cancers, adenocarcinoma, cancer of the pancreas, stomach, ovaries, prostate, colon, lung, brain, breast, kidney, skin, cervix, and ear- nose- throat cancer.

"Vitamin B3" mentioned herein includes vitamin B3 in its various forms, including niacinamide, nicotinic acid, nicotinamide, inositol hexaniacinate.

"Vitamin C" mentioned herein includes vitamin C (i.e., L-ascorbic acid, D-ascorbic acid, or both) and its salts (e.g., sodium ascorbate).

"Folic acid" mentioned herein includes vitamin B9, folate, pteroylglutamic acid, 5-L-5-methyl tetrahydro folate, and L-methyl folate.

The term "improve" is used to convey that the compounds or methods of embodiments herein change either the appearance, form, characteristics and/or the physical attributes of the condition or tissue to which it is being provided, applied or administered.

The terms "improving," "treating," and "reducing" refer to the administration of an effective amount of an isoquercetin, quercetin or rutin composition of the invention to a subject, who needs to improve one or more of the above-mentioned conditions or has one or more of the just-mentioned disorders, or a symptom or a predisposition of one of more of the disorders or conditions, with the purpose to improve one or more of these conditions, or to prevent, cure, alleviate, relieve, remedy, or ameliorate one or more of these disorders, or the symptoms or the predispositions of one or more of them. The term "administration" covers oral or parenteral delivery to a subject the quercetin, isoquercetin, or rutin composition (or any suitable derivative thereof) of the invention in any suitable form, e.g., food product, beverage, tablet, capsule, suspension, and sterile injectable solution. The term "parenteral" refers to subcutaneous, intracutaneous, intravenous, intramuscular, intraarticular, intraarterial, intrasynovial, intrasternal, intrathecal, intralesional, and intracranial injection, as well as various infusion techniques. An "effective amount" refers to a dose of the isoquercetin, quercetin, or rutin composition that is sufficient to provide a therapeutic benefit (e.g., reducing the levels of PDI activity in the serum and/or soluble P selectin in the patient in need thereof, e.g. a cancer patient with elevated levels of soluble P selectin). In certain embodiments, an effective amount of isoquercetin is about 1000mg. In certain embodiments, the effective amount of isoquercetin can range from about 1,000mg-2,000mg. In additional embodiments an effective amount of isoquercetin ranges from about 2,000mg-2,500mg. A particularly preferred effective amount of isoquercetin is 1000mg.

### Methods of Treatment (the methods are to be interpreted as references to compounds, pharmaceutical compositions and medicaments of the present invention for use in those methods)

Thrombosis is a common complication of advanced stage cancer, including advanced solid tumor cancers, as well as advanced blood cancers. However, the underlying mechanisms that link tumor progression to clot formation are poorly understood.

The present invention relates to methods for identifying and/or monitoring risk of a thrombotic condition in a cancer patient, particularly in patients with advanced cancer. Some embodiments of the invention relate to methods for identifying and/or monitoring virally induced thrombotic conditions, genetically induced thrombotic conditions, or anemia induced thrombotic conditions in a patient. The present invention also relates to an effective amount of isoquercetin and optionally an antithrombotic agent for use in a method of treating a thrombotic condition in a cancer patient, wherein the cancer patient has been diagnosed as being at risk of a thrombotic event by a method of the present invention.

Thrombus formation involves several sequential steps that typically begin following a skin laceration or a vascular injury. Circulating platelets first come into proximity to the site of injured endothelial cells and a series of events occurs that allows activation of these platelets. Activated platelets then recruit additional platelets to the site of injury, where they aggregate to form a plug until a stable clot forms. Inactive coagulation factors, which are always present and circulating in the bloodstream, are then sequentially activated in a process known as the coagulation cascade. The coagulation cascade ultimately leads to a stable fibrin-containing clot.

Thrombotic disorders are a group of inherited and acquired disorders that cause abnormal activation of the hemostatic system, leading to an increased risk of venous and arterial thrombosis. Cancer is among the acquired disorders that greatly increase the risk of thrombosis. Tumor cells, by expressing high levels of tissue factor on their surface, cause a hypercoagulable state. Tissue factor is required for initiating the just-mentioned coagulation cascade.

Among the factors involved in thrombus formation is protein disulfide isomerase (PDI). PDI is leaks from activated endothelial cells and platelets, after which it plays a critical role in thrombus formation. PDI can activate tissue factor, which leads to activation of the coagulation cascade, ultimately resulting in fibrin deposition and thrombus formation.

Protein disulfide isomerase is a thiol isomerase that is primarily localized to the endoplasmic reticulum where it serves an essential role in protein folding. However, PDI can also be released from cells in disease states or following tissue injury and contribute to pathological processes. PDI has been implicated in cancer, neurodegenerative disease, infectious disease, and thromboembolism. In the context of thromboembolic disease, PDI is released from activated platelets and endothelial cells and is postulated to modulate through oxidation, reduction, or isomerization a number of extracellular coagulation substrates as factor XI, tissue factor, factor V, vitronectin, αIIbβ3, and αVβ3. Targeting PDI activity with blocking antibodies or small molecules prevents both platelet accumulation and fibrin generation at the site of vascular injury in several distinct animal models of thrombosis.

Additional methods and compositions for identifying patients at risk as well as preventing and reducing venous or arterial thrombotic events are needed, particularly in patients with advanced cancer, including solid tumor cancers and blood cancers.

Thrombosis is a common complication of advanced stage cancer, including advanced solid tumor cancers, as well as advanced blood cancers. However, the underlying mechanisms that link tumor progression to clot formation are poorly understood.

While the various embodiments herein refer to cancer patients, the patient can also be a patient that does not have cancer. In some embodiments the patient has a virally induced thrombotic condition, genetically induced thrombotic condition, or anemia induced thrombotic condition.

Some embodiments of the present invention describe an effective amount of isoquercetin and optionally an antithrombotic agent for use in a method of treatment of a thrombotic condition in a cancer patient, wherein the cancer patient has been identified as being at-risk for a thrombotic event when a plasma sample from the patient exhibits elevated levels of the UPR biomarkers PPIA, PDIA3, and at least one of EIF5A, EIF4H, EIF4a3, UBE2N, UBE2L3, UBE2I, and HSP70 (which are elevated in plasma samples from patients with advanced cancer that later exhibited VTE, as compared to non-elevated levels of the same UPR biomarker proteins in plasma samples from patients with advanced cancer that did not exhibit VTE, and as such serve as the baseline reference samples). In certain embodiments, the cancer patient is one who is actively undergoing treatment for cancer including receiving chemotherapeutic treatments and/or radiation, and/or immunotherapy, and/or cell therapy.

Some embodiments of the present invention describe an effective amount of an isoquercetin for use in a method of reducing or preventing formation of a thrombus in the at-risk patient with cancer, wherein the patient has been identified as at-risk for a thrombotic event by detecting in a plasma sample from the patient elevated levels of the UPR biomarkers PPIA, PDIA3 and at least one of EIF5A, EIF4H, EIF4a3, UBE2N, UBE2L3, UBE2I, and HSP70, (which are elevated in plasma samples from patients with advanced cancer that later exhibited VTE, as compared to non-elevated levels of the same UPR biomarker proteins in plasma samples from patients with advanced cancer that did not exhibit VTE, and as such serve as the baseline reference samples). In certain embodiments, the cancer patient is one who is actively undergoing treatment for cancer including receiving chemotherapeutic treatments and/or radiation, and/or immunotherapy, and/or cell therapy.

In certain embodiments, a sample of biological tissue or bodily fluid from a subject with cancer is obtained. A protein sample can be obtained from any biological tissue. Examples of biological tissues include, but are not limited to, epidermal, whole blood, and plasma. A protein sample can also be obtained from any biological fluid. Examples of fluids include, but are not limited to, plasma, saliva, and urine.

In some embodiments, according to any method described herein, the patient exhibits no severe adverse events (grade 3 or 4 toxicities) during treatment.

In some embodiments, according to any method described herein, the patient exhibits no primary venous thromboembolism (VTE) during treatment.

In some embodiments, according to any method described herein, the patient exhibits no VTE for at least 30-60 days following treatment.

In some embodiments, according to any method described herein, the patient exhibits no major hemorrhages during treatment.

### Rutin, Quercetin, Isoquercetin and Related Derivatives

The terms "isoquercetin" "quercetin" and "rutin" refer to certain active compounds for administration.

Isoquercetin (2-(3,4-Dihydroxyphenyl)-5,7-dihydroxy-3-[(2*S*,3*R*,4*S*,5*S*,6*R*)-3,4,5-trihydroxy-6-(hydroxymethyl)oxan-2-yl]oxychromen-4-one) is a 3-O-glucoside of quercetin having the following structure:

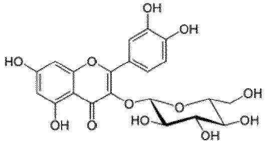

Rutin (2-(3,4-dihydroxyphenyl)-5,7-dihydroxy-3-[α-L-rhamnopyranosyl-(1→6)-β-D-glucopyranosyloxy]-4*H*-chromen-4-one) is another common glycoside that has disaccharide rutinose (α-L-rhamopyranosyl-(1→ 6 - *β - D* - glucopyranose) attached at the 3 O position of the quercetin having the following structure:

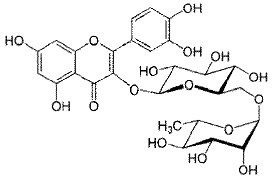

Quercetin is characterized by the following structure:

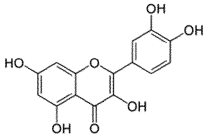

Quercetin or quercetin derivatives may include quercetin-5-O-glucoside, quercetin-7-O-glucoside, quercetin-9-O-glucoside, quercetin-3-O-[.alpha.-rhamnosyl-(1.fwdarw.2)-.alpha.-rhamnosyl-(1.fwdarw- .6)]-.beta.-glucoside, quercetin-3-O-galactoside, quercetin-7-O-galactoside, quercetin-3-O-rhamnoside, isoquercetin, rutin, and quercetin-7-O-galactoside. After digestion, quercetin derivatives are converted in the body to quercetin aglycon and/or other active derivatives, including methylated, sulphated and gluconorated forms which are absorbed in the body.

In some embodiments described herein the compound for use in the present methods is isoquercetin. Suitable conjugates or derivatives include methylates, sulfates and glucoronides.

In any embodiment described herein, the isoquercetin can be added to the composition either in a pure form or as an ingredient in a mixture (e.g., a plant extract). Examples of commercially available quercetin include QU995 (containing 99.5% quercetin) and QU985 (containing 98.5% quercetin) from Quercegen Pharmaceuticals LLC (Boston, Mass.). Examples of commercially available isoquercetin compounds include those available from Quercegen Pharmaceuticals LLC: ISQ 995 AN (99.5% pure all-natural isoquercetin) and ISQ 995 CIT (99.5% pure isoquercitrin). Additional methods and isoquercetin compositions can be found in U.S. Pat. Nos. 7,745,486 and 7,745,487.

The isoquercetin according to any embodiment described herein may be administered by oral or parenteral (e.g., intramuscular, intraperitoneal, intravenous, ICV, intracisternal injection or infusion, subcutaneous injection, or implant) dosage form and may be formulated, alone or together, in suitable dosage unit formulations containing conventional nontoxic pharmaceutically acceptable carriers, adjuvants and vehicles appropriate for each route of administration. The compounds and compositions described herein may also be formulated as a controlled-release formulation.

The isoquercetin compositions according to any embodiment described herein can be administered in a wide range of dosage-forms including, for example, solid dosage forms and liquid dosage forms. Solid dosage forms may include powders, tablets, pills, capsules, suppositories, or dispersible granules. A solid carrier can be one or more substances that function as a diluting agent, flavor additive, solvent, lubricant, suspension agent, binder, preservative, tablet-disintegrating substance or encapsulating material. In powdered form, the carrier may be a finely pulverized solid including lactose, hydroxypropylmethylcellulose and PVP, mixed with an appropriate amount of the active ingredient. Appropriate carriers for powder and tablet forms include for example magnesium carbonate, magnesium stearate, talc, sugar, lactose, pectin, dextrin, stiffeners, gelatins, tragacanth, methylcellulose, and sodium carboxymethylcellulose.

Liquid dosage forms include for example solutions, suspensions, and emulsions. Also included are compositions in solid form that are meant to be converted to liquid form shortly prior to consumption. These forms may include, in addition to the active ingredients, artificial colors, flavors, stabilizers, buffers, natural or artificial sweeteners, dispersing agents, thickeners, dissolving agents and the like.

Solutions or mixtures may be administered directly to the nasal cavity using conventional means, such as drops or sprays. The composition may be produced in individual or multi-dose forms. Multi-dose forms would include a dropper, pipette or atomizer that delivers a predetermined volume of the composition.

The isoquercetin according to any embodiment described herein may be provided in individual dosage units that contain a suitable amount of the active ingredient.

The individual doses may be provided in a package, or as a kit that includes a measuring device, e.g., a device for measuring oral or injectable dosages (i.e., a measuring cup, needle, or syringe). The kit can also include, other materials such buffers, diluents, filters, and package inserts with instructions for use. A label may be present on the on the kit to indicate that the composition is used for a specific therapy, and may also indicate directions for use.

If desired, the compositions for use as defined in the present invention may further comprise one or more additional active agents. Where it is appropriate, any of the active agents may be administered in the form of the compound per se, and/or in the form of a salt, polymorph, ester, amide, prodrug, derivative, or the like, provided the salt, polymorph, ester, amide, prodrug or derivative is suitable pharmacologically. Where it is appropriate, salts, esters, amides, prodrugs and other derivatives of the active agents may be prepared using standard procedures known to those skilled in the art of synthetic organic chemistry and described, for example, by J. March, Advanced Organic Chemistry: Reactions, Mechanisms and Structure, 4th Ed. (New York: Wiley-Interscience, 1992). For any active agents that may exist in enantiomeric forms, the active agent may be incorporated into the present compositions either as the racemate or in enantiomerically enriched form.

In some embodiments the dosage of the isoquercetin compositionsbeing administered will depend on the condition being treated, the particular compound, and other clinical factors such as age, sex, weight, and health of the subject being treated, the route of administration of the compound(s), and the type of composition being administered (tablet, gel cap, capsule, solution, suspension, inhaler, aerosol, elixir, lozenge, injection, patch, ointment, cream, etc.). It is to be understood that the present disclosure has application for both human and animal use. The amount of the isoquercetin according to any embodiment described, required for use in treatment will be ultimately at the discretion of the attendant physician or clinician.

In some embodiments, the isoquercetin composition, or any suitable derivative thereof, can be in a soft chew composition that includes isoquercetin, quercetin, or rutin or any suitable derivative thereof, niacinamide, ascorbic acid, sodium ascorbate, folic acid, sugar, corn syrup, sucralose, soy lecithin, sunflower lecithin, corn starch, glycerin, palm oil, xylitol, carrageenan, FD&C Yellow #6, FD&C Yellow #5, or natural or artificial flavors. Optionally, isoquercetin compositions described herein can further comprise components such as vitamin B₃, vitamin C and or folic acid. An exemplary soft chew composition (5.15 g) includes 250 mg of isoquercetin, 12.9 mg of vitamin B₃ (i.e., niacinamide), and 382.8 mg of vitamin C (i.e., L-ascorbic acid and sodium ascorbate). In further exemplary embodiments, the components of the exemplary soft chew are the same, except the active agent is replaced with 500 mg or 1000 mg of isoquercetin. For example, a subject can take one to eight servings (e.g., 4 servings) of this soft chew composition daily. The amounts taken can vary depending on, for example, the disorder or condition to be treated and the physical states of the subject. Another exemplary composition of this soft chew includes 5.25 wt % of quercetin, 0.25 wt % of vitamin B₃, and 7.81 wt % of vitamin C (i.e., L-ascorbic acid and sodium ascorbate) plus 200 µg of folic acid per chew.

In some embodiments the isoquercetin is administered in a composition comprising Vitamin B3, and optionally further comprises Vitamin C, and further optionally comprises folic acid.

In some embodiments the isoquercetin is administered in a composition comprising about 20 micrograms to about 3 grams of Vitamin B3, and optionally further comprises about 200 micrograms to about 3 grams of Vitamin C, and further optionally comprises 1000 micrograms to about 3000 micrograms of folic acid (e.g. folate).

When the above-described composition is in powder form, it can be used conveniently to prepare beverage, paste, jelly, capsules, or tablets. Lactose and corn starch are commonly used as diluents for capsules and as carriers for tablets. Lubricating agents, such as magnesium stearate, are typically included in tablets.

The oral bioavailability of isoquercetin in the above-mentioned capsule or tablet formulations can be improved by the use of certain additives. For example, a capsule or tablet can include acid treated gelatin, citrate, potassium hydroxide, and/or a cyclodextrin. A preferred amount of these additives per mg of isoquercetin, quercetin, or rutin is 0.01-0.5 mg potassium hydroxide, 0.01-0.7 mg acid treated gelatin, 0.1-1 mg citrate, and 0.01-1 mg of a cyclodextrin. Isoquercetin, quercetin, or rutin, in the presence of the additives, can have a solubility in an aqueous solution of 2-5%. Additionally, the pH of a isoquercetin -containing formulation with improved oral bioavailability can be between pH 7 and pH 12.

The isoquercetin composition may be administered as a dietary supplement or a pharmaceutical formulation. As a dietary supplement, additional nutrients, such as minerals or amino acids may be included. A pharmaceutical formulation can be a sterile injectable or infusible solution that contains the isoquercetin, quercetin, or rutin composition together with pharmaceutically acceptable excipients. The isoquercetin composition can also be a food product. As used herein, the term "food" broadly refers to any kinds of liquid and solid/semi-solid materials that are used for nourishing humans and animals, for sustaining normal or accelerated growth, or for maintaining stamina or alertness. Examples of human food products include, but are not limited to, tea-based beverages, juice, coffee, milk, jelly, cookies, cereals, chocolates, snack bars, herbal extracts, dairy products (e.g., ice cream, and yogurt), soy bean product (e.g., tofu), and rice products.

The dosage of the compound as an active ingredient in the compositions of this invention may be varied so that a suitable dosage form is obtained. The active ingredient may be administered to patients (animals and human) in need of such treatment in dosages that will provide optimal pharmaceutical efficacy. The selected dosage depends upon the desired therapeutic effect, on the route of administration, and on the duration of the treatment. The dose will vary from patient to patient depending upon the nature and severity of disease, the patient's weight, special diets then being followed by a patient, concurrent medication, and other factors which those skilled in the art will recognize.

In some embodiments, the therapeutically effective amount will be about 500 mg to up to 5 grams daily. In certain additional embodiments, the therapeutically effective amount will be about 4 grams, or 3 grams, or even 2 grams. In certain embodiments, the therapeutically effective amount will be about 500 mg to about 2000 mg daily.

In some embodiments the therapeutically effective amount is between a lower limit of about 500 mg/day, about 525 mg/day, about 550 mg/day, about 575 mg/day, about 600 mg/day, about 625 mg/day, about 650 mg/day, about 675 mg/day, about 700 mg/day, about 725 mg/day, about 750 mg/day, about 775 mg/day, about 800 mg/day, about 825 mg/day, about 850 mg/day, about 875 mg/day, about 900 mg/day, about 925 mg/day, about 950 mg/day, about 975 mg/day, about 1000 mg/day, about 1025 mg/day, about 1050 mg/day, about 1075 mg/day, about 1100 mg/day, 1125 mg/day, about 1150 mg/day, about 1175 mg/day, about 1200 mg/day, 1225 mg/day, about 1250 mg/day, about 1275 mg/day, about 1300 mg/day, 1325 mg/day, about 1350 mg/day, about 1375 mg/day, about 1400 mg/day, 1425 mg/day, about 1450 mg/day, about 1475 mg/day, about 1500 mg/day, about 1525 mg/day, about 1550 mg/day, about 1575 mg/day, about 1600 mg/day, about 1625 mg/day, about 1650 mg/day, about 1675 mg/day, about 1700 mg/day, about 1725 mg/day, about 1750 mg/day, about 1775 mg/day, about 1800 mg/day, about 1825 mg/day, about 1850 mg/day, about 1875 mg/day, about 1900 mg/day, about 1925 mg/day, about 1950 mg/day, about 1975 mg/day, about 2000 mg/day, about 2025 mg/day, about 2050 mg/day, about 2075 mg/day, about 2100 mg/day, 2125 mg/day, about 2150 mg/day, about 2175 mg/day, about 2200 mg/day, 2225 mg/day, about 2250 mg/day, about 2275 mg/day, about 2300 mg/day, 2325 mg/day, about 2350 mg/day, about 2375 mg/day, about 2400 mg/day, 2425 mg/day, about 2450 mg/day, about 2475 mg/day, about 2500 mg/day, about 2525 mg/day, about 2550 mg/day, about 2575 mg/day, about 2600 mg/day, about 2625 mg/day, about 2650 mg/day, about 2675 mg/day, about 2700 mg/day, about 2725 mg/day, about 2750 mg/day, about 2775 mg/day, about 2800 mg/day, about 2825 mg/day, about 2850 mg/day, about 2875 mg/day, about 2900 mg/day, about 2925 mg/day, about 2950 mg/day, about 2975 mg/day, about 3000 mg/day, about 3025 mg/day, about 3050 mg/day, about 3075 mg/day, about 3100 mg/day, 3125 mg/day, about 3150 mg/day, about 3175 mg/day, about 3200 mg/day, 3225 mg/day, about 3250 mg/day, about 3275 mg/day, about 3300 mg/day, 3325 mg/day, about 3350 mg/day, about 3375 mg/day, about 3400 mg/day, 3425 mg/day, about 3450 mg/day, about 3475 mg/day, about 3500 mg/day, about 3525 mg/day, about 3550 mg/day, about 3575 mg/day, about 3600 mg/day, about 3625 mg/day, about 3650 mg/day, about 3675 mg/day, about 3700 mg/day, about 3725 mg/day, about 3750 mg/day, about 3775 mg/day, about 3800 mg/day, about 3825 mg/day, about 3850 mg/day, about 3875 mg/day, about 3900 mg/day, about 3925 mg/day, about 3950 mg/day, about 3975 mg/day, about 4000 mg/day, about 4025 mg/day, about 4050 mg/day, about 4075 mg/day, about 4100 mg/day, 4125 mg/day, about 4150 mg/day, about 4175 mg/day, about 4200 mg/day, 4225 mg/day, about 4250 mg/day, about 4275 mg/day, about 4300 mg/day, 4325 mg/day, about 4350 mg/day, about 4375 mg/day, about 4400 mg/day, 4425 mg/day, about 4450 mg/day, about 4475 mg/day, about 4500 mg/day, about 4525 mg/day, about 4550 mg/day, about 4575 mg/day, about 4600 mg/day, about 4625 mg/day, about 4650 mg/day, about 4675 mg/day, about 4700 mg/day, about 4725 mg/day, about 4750 mg/day, about 4775 mg/day, about 4800 mg/day, about 4825 mg/day, about 4850 mg/day, about 4875 mg/day, about 4900 mg/day, about 4925 mg/day, about 4950 mg/day, about 4975 mg/day, and about 5000 mg/day; and an upper limit of about 5000 mg/day, about 4975 mg/day, about 4950 mg/day, about 4925 mg/day, about 4900 mg/day, about 4875 mg/day, about 4850 mg/day, about 4825 mg/day, about 4800 mg/day, about 4775 mg/day, about 4750 mg/day, about 4725 mg/day, about 4700 mg/day, about 4675 mg/day, about 4650 mg/day, about 4625 mg/day, about 4600 mg/day, about 4575 mg/day, about 4550 mg/day, about 4525 mg/day, about 4500 mg/day, 4475 mg/day, about 4450 mg/day, about 4425 mg/day, about 4400 mg/day, about 4375 mg/day, about 4350 mg/day, about 4325 mg/day, about 4300 mg/day, about 4275 mg/day, about 4250 mg/day, about 4225 mg/day, about 4200 mg/day, about 4175 mg/day, about 4150 mg/day, about 4125 mg/day, about 4100 mg/day, about 4075 mg/day, about 4050 mg/day, about 4025 mg/day, about 4000 mg/day, 3975 mg/day, about 3950 mg/day, about 3925 mg/day, about 3900 mg/day, about 3875 mg/day, about 3850 mg/day, about 3825 mg/day, about 3800 mg/day, about 3775 mg/day, about 3750 mg/day, about 3725 mg/day, about 3700 mg/day, about 3675 mg/day, about 3650 mg/day, about 3625 mg/day, about 3600 mg/day, about 3575 mg/day, about 3550 mg/day, about 3525 mg/day, about 3500 mg/day, 3475 mg/day, about 3450 mg/day, about 3425 mg/day, about 3400 mg/day, about 3375 mg/day, about 3350 mg/day, about 3325 mg/day, about 3300 mg/day, about 3275 mg/day, about 3250 mg/day, about 3225 mg/day, about 3200 mg/day, about 3175 mg/day, about 3150 mg/day, about 3125 mg/day, about 3100 mg/day, about 3075 mg/day, about 3050 mg/day, about 3025 mg/day, about 3000 mg/day, 2975 mg/day, about 2950 mg/day, about 2925 mg/day, about 2900 mg/day, about 2875 mg/day, about 2850 mg/day, about 2825 mg/day, about 2800 mg/day, about 2775 mg/day, about 2750 mg/day, about 2725 mg/day, about 2700 mg/day, about 2675 mg/day, about 2650 mg/day, about 2625 mg/day, about 2600 mg/day, about 2575 mg/day, about 2550 mg/day, about 2525 mg/day, about 2500 mg/day, 2475 mg/day, about 2450 mg/day, about 2425 mg/day, about 2400 mg/day, about 2375 mg/day, about 2350 mg/day, about 2325 mg/day, about 2300 mg/day, about 2275 mg/day, about 2250 mg/day, about 2225 mg/day, about 2200 mg/day, about 2175 mg/day, about 2150 mg/day, about 2125 mg/day, about 2100 mg/day, about 2075 mg/day, about 2050 mg/day, about 2025 mg/day, about 2000 mg/day, 1975 mg/day, about 1950 mg/day, about 1925 mg/day, about 1900 mg/day, about 1875 mg/day, about 1850 mg/day, about 1825 mg/day, about 1800 mg/day, about 1775 mg/day, about 1750 mg/day, about 1725 mg/day, about 1700 mg/day, about 1675 mg/day, about 1650 mg/day, about 1625 mg/day, about 1600 mg/day, about 1575 mg/day, about 1550 mg/day, about 1525 mg/day, about 1500 mg/day, 1475 mg/day, about 1450 mg/day, about 1425 mg/day, about 1400 mg/day, about 1375 mg/day, about 1350 mg/day, about 1325 mg/day, about 1300 mg/day, about 1275 mg/day, about 1250 mg/day, about 1225 mg/day, about 1200 mg/day, about 1175 mg/day, about 1150 mg/day, about 1125 mg/day, about 1100 mg/day, about 1075 mg/day, about 1050 mg/day, about 1025 mg/day, about 1000 mg/day, about 975 mg/day, about 950 mg/day, about 925 mg/day, about 900 mg/day, about 875 mg/day, about 850 mg/day, about 825 mg/day, about 800 mg/day, about 775 mg/day, about 750 mg/day, about 725 mg/day, about 700 mg/day, about 675 mg/day, about 650 mg/day, about 625 mg/day, about 600 mg/day, about 575 mg/day, about 550 mg/day, about 525 mg/day, and about 500 mg/day

The compounds may be administered on a regimen of 1 to 4 times per day, such as once, twice, three times or four times per day.

The efficacy of administering isoquercetin to reduce the hypercoagulability in cancer patients was evaluated (*See,* Zwicker et al., JCI Insight.2019; 4(4):e125851, and also Clinicaltrials.gov NCT02195232). Venous thromboembolism (VTE) is commonly observed in cancer patients and is a leading cause of mortality in this population. In high risk cancer patients especially where protocol-driven radiographic monitoring for deep vein thrombosis is implemented, the incidence of VTE within initial few months of chemotherapy commonly exceeds 20%. Cancer patients are also at an increased risk of bleeding which has limited the adoption of routine primary thromboprophylaxis in cancer outpatients receiving chemotherapy. Developing diagnostic and treatment methods that reduce the incidence of VTE without increasing the risk of major hemorrhage would broadly impact the care of patients with advanced malignancy, as well as any patient with cancer.

As used herein, the types of cancer can be selected from the group consisting of estrogen receptor-dependent breast cancer, estrogen receptor-independent breast cancer, hormone receptor-dependent prostate cancer, hormone receptor-independent prostate cancer, brain cancer, renal cancer, glioblastoma, colon cancer, familial adenomatous polyposis (FAP), colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis; ichtiosis; acne, acne vulgaris, sarcomas, Kaposi's sarcoma, osteosarcoma, head and neck cancer, small cell lung carcinoma, non-small cell lung carcinoma, leukemia, lymphomas and/or other blood cell cancers.

Additional cancers that will benefit from the methods described herein include cancers associated with certain viruses (and include improving a pre-cancerous condition during viral infection). Such conditions include those associated with Human T-cell leukemia virus type, also called human T-lymphotrophic virus (HTLV-1) which is linked to adult T-cell leukemia/lymphoma. Another such cancer include those associated with human papillomavirus (HPV), which has at least 12 strains that can cause cancer in men and women, including anal, cervical, penile, throat, vaginal and vulvar cancer. Additional condition includes those associated with human herpes virus 8 (HHV-8), which is associated with Kaposi sarcoma in people who have a weakened immune system (e.g. patients with HIV). Similarly, there are numerous cancers associated with HIV, which is believed to damage the immune system and reduce defenses against other oncoviruses. HIV-associated cancers include Kaposi sarcoma, non-Hodgkin's and Hodgkin's lymphoma, cervical cancer, and cancers of the anus, liver, mouth and throat and lung. Additionally, hepatitis C is a leading cause of liver cancer, and can cause non-Hodgkin's lymphoma, and as such can benefit from the methods described herein. Similarly, hepatitis B is a leading cause of liver cancer, and these conditions can benefit from the methods described herein. Finally, Epstein-Barr virus (EBV) infection increases the risk of Burkitt lymphoma, some types of Hodgkin's and non-Hodgkin's lymphoma and stomach cancer, and these conditions can also benefit from the methods described herein.

In certain embodiments, the cancer is a metastasizing cancer. A "metastasizing cancer" is a cancer which may form or often forms metastases. A metastasizing cancer which has already spread from the part of the body where it started, i.e. the primary site, to other parts of the body, is also denoted metastatic cancer. When cancer cells break away from a tumor, they can travel to other areas of the body through the bloodstream or the lymph system. Such cancer cells may then form new tumors in other areas of the body.

In certain embodiments, the cancer is a metastasizing cancer selected from the group consisting of metastasizing forms of Hodgkin lymphoma, colorectal cancer, cervical cancer, lung cancer, skin cancer such as squamous cell cancer or basal cell carcinoma, head and neck cancer, gastric cancer, pancreatic cancer, head and neck squamous cell cancer, and breast cancer.

In some embodiments the metastatic cancer is colorectal cancer, pancreatic cancer, or non-small cell lung cancer.

In certain embodiments, the cancer is classifiable as Stage III or Stage IV according to the TNM anatomic/prognostic group system of the cancer staging system of the American Joint Committee on Cancer. In additional embodiments, the cancer is classifiable as Stage IV according to the TNM anatomic/prognostic group system of the cancer staging system of the American Joint Committee on Cancer.

In certain embodiments, the cancer is a metastasizing cancer selected from the group consisting of metastasizing forms of Hodgkin lymphoma, colorectal cancer, cervical cancer, lung cancer, skin cancer such as squamous cell cancer or basal cell carcinoma, head and neck cancer, gastric cancer, pancreatic cancer, and breast cancer, wherein said metastasizing cancer is classifiable as Stage IV according to the TNM anatomic/prognostic group system of the cancer staging system of the American Joint Committee on Cancer (7.sup.th edition, 2010, Springer).

In certain embodiments, the isoquercetin composition is used in combination with the detection of the UPR biomarker panel for decreasing or preventing thrombotic conditions and for use in combination with other treatments for treating cancers in patients with cancers, including those with metastases that have already formed, such as metastasizing forms of Hodgkin lymphoma, colorectal cancer, cervical cancer, head and neck cancer, gastric cancer, non-small cell lung cancer, pancreatic cancer and breast cancer in a mammalian, typically human subject. In additional embodiments, the isoquercetin composition is used in patients without metastatic cancer, but who exhibit cancer only at a primary site. Additionally, it is expected that the methods and treatments described herein will be effective in treating any solid or blood cell cancer, since all patients with these cancers, whether or not they are metastatic, will benefit from reduced levels of plasma PDI and/or soluble P-selectin, and furthermore will benefit from the diagnosing, monitoring, decreasing or eliminating venous thromboembolisms (VTE's), or other thrombotic conditions, without increasing the risk of major hemorrhage. It is noted that patients with cancer typically exhibit high levels of soluble P selectin, and as a result, are at an elevated risk for developing venous thromboembolisms (VTE's) and related thrombotic conditions. Thus, in certain embodiments, a combination of the UPR biomarker panel along with elevated levels of soluble P selectin may be a useful screening tool for identifying cancer patients at risk for thrombotic events, for whom prophylactic treatment would be beneficial.

In a preferred embodiment of the invention, the reagents that specifically bind to the UPR biomarker proteins PPIA, EIF5A, EIF4H, EIF4a3, UBE2N, UBE2L3, UBE2I, HSP70, and PDIA3 (which are elevated in plasma samples from patients with advanced cancer that later exhibited VTE, as compared to non-elevated levels of the same UPR biomarker proteins in plasma samples from patients with advanced cancer that did not exhibit VTE, and as such serve as the baseline reference samples) or any subset or combination thereof, and to the optionally one or more other biomarkers are immobilized on a solid support such as for example a polystyrene surface. A solid support may suitably be a protein microarray or protein array device for the simultaneous binding and quantification of the marker panel used to assess risk of thrombotic conditions. The protein array device consists of molecules (capture agents) bound to a defined spot position on a support material. Preferably biotinylated specific binding reagents are bound as very small spots onto a solid phase that is coated with streptavidin. The array is then exposed to the sample. Capture agents such as antibodies are able to bind the protein of interest from the biological sample. The binding of the specific analyte proteins to the individual spots can then be monitored by quantifying the signal generated by each spot.

In another preferred embodiment of the invention the reagents that specifically bind to UPR biomarker proteins PPIA, PDIA3 and at least one of EIF5A, EIF4H, EIF4a3, UBE2N, UBE2L3, UBE2I, and HSP70 (which are elevated in plasma samples from patients with advanced cancer that later exhibited VTE, as compared to non-elevated levels of the same UPR biomarker proteins in plasma samples from patients with advanced cancer that did not exhibit VTE, and as such serve as the baseline reference samples), and to the optionally one or more other biomarkers are immobilized on a solid support such as for example a polystyrene surface. A solid support may suitably be a protein microarray or protein array device for the simultaneous binding and quantification of the marker panel used to assess risk of thrombotic conditions. The protein array device consists of molecules (capture agents) bound to a defined spot position on a support material. Preferably biotinylated specific binding reagents are bound as very small spots onto a solid phase that is coated with streptavidin. The array is then exposed to the sample. Capture agents such as antibodies are able to bind the protein of interest from the biological sample. The binding of the specific analyte proteins to the individual spots can then be monitored by quantifying the signal generated by each spot.

A protein array device comprising at least the appropriate specific binding partners for measurement of UPR biomarker expression levels and optionally appropriate specific binding partners for one or more other marker useful in assessing the risk of thrombotic conditions in a patient with cancer, and in particular a patient with advanced cancer.

Suitable immunoassays for detecting protein expression level in a plasma sample that are commonly employed in the art include, for example and without limitation, western blot, immunoprecipitation, enzyme-linked immunosorbent assay (ELISA), radioimmunoassay (RIA), fluorescent activated cell sorting (FACS), immunoradiometric assay, gel diffusion precipitation reaction, immunodiffusion assay, *in situ* immunoassay, imaging mass cytometry, complement fixation assay, and immunoelectrophoresis assay. In accordance with this aspect of the disclosure, the measured UPR biomarker expression levels in the patient sample (from a cancer patient) can further be compared to the UPR biomarker protein expression level measured in a baseline, reference, or control sample, e.g., levels of PPIA, PDIA3 and at ;east one of EIF5A, EIF4H, EIF4a3, UBE2N, UBE2L3, UBE2I, and HSP70 and optionally P selectin from a cancer patient who does not exhibit VTE or other thrombotic conditions for at least 8 weeks; and diagnosing the patient as at risk of a thrombotic event when PPIA, PDIA3 and at least one of EIF5A, EIF4H, EIF4a3, UBE2N, UBE2L3, UBE2I, and HSP70, and optionally P selectin are elevated over the baseline, control, or reference level.

In another embodiment, the UPR biomarker expression levels are measured using one-dimensional and two-dimensional electrophoretic gel analysis, high performance liquid chromatography (HPLC), reverse phase HPLC, Fast protein liquid chromatograph (FPLC), mass spectrometry (MS), tandem mass spectrometry, liquid crystal-MS (LC-MS) surface enhanced laser desorption/ionization (SELDI), MALDI, and/or protein sequencing.

Described herein but not claimed, the UPR biomarker expression levels, particularly in plasma samples, can also or alternatively be measured by detecting and quantifying the corresponding UPR biomarker panel nucleic acid levels using a nucleic acid detection assay. In one embodiment, RNA, e.g., mRNA, levels are measured. RNA is preferably reverse-transcribed to synthesize complementary DNA (cDNA), which is then amplified and detected or directly detected. The detected cDNA is measured and the levels of cDNA serve as an indicator of the RNA or mRNA levels present in a sample. Reverse transcription may be performed alone or in combination with an amplification step, e.g., reverse transcription polymerase chain reaction (RT-PCR), which may be further modified to be quantitative, e.g., quantitative RT-PCR as described in U.S. Patent No. 5,639,606.

It may be beneficial or otherwise desirable to extract RNA from the plasma sample prior to or for analysis. RNA molecules can be isolated from the sample and the concentration (i.e., total RNA) quantified using any number of procedures, which are well-known in the art, the particular extraction procedure chosen based on the particular biological sample. In some instances, with some techniques, it may also be possible to analyze the nucleic acid without extraction from the sample.

mRNA can be analyzed directly without an amplification step. Direct analysis may be performed with different methods including, but not limited to, nanostring technology (Geiss et al. "Direct Multiplexed Measurement of Gene Expression with Color-Coded Probe Pairs," Nat Biotechnol 26(3): 317-25 (2008)). Nanostring technology enables identification and quantification of individual target molecules in a biological sample by attaching a color coded fluorescent reporter to each target molecule. This approach is similar to the concept of measuring inventory by scanning barcodes. Reporters can be made with hundreds or even thousands of different codes allowing for highly multiplexed analysis. Direct analysis can be performed using immunohistochemical techniques.

It may be beneficial or otherwise desirable to reverse transcribe and amplify the RNA prior to detection/analysis. Methods of nucleic acid amplification, including quantitative amplification, are commonly used and generally known in the art. Quantitative amplification will allow quantitative determination of relative amounts of RNA in the cells.

Nucleic acid amplification methods include, without limitation, polymerase chain reaction (PCR) (U.S. Pat. No. 5,219,727) and its variants such as in situ polymerase chain reaction (U.S. Pat. No. 5,538,871), quantitative polymerase chain reaction (U.S. Pat. No. 5,219,727), nested polymerase chain reaction (U.S. Pat. No. 5,556,773), self-sustained sequence replication and its variants (Guatelli et al. "Isothermal, In vitro Amplification of Nucleic Acids by a Multienzyme Reaction Modeled after Retroviral Replication," Proc Natl Acad Sci USA 87(5): 1874-8 (1990), transcriptional amplification and its variants (Kwoh et al. "Transcription-based Amplification System and Detection of Amplified Human Immunodeficiency Virus type 1 with a Bead-Based Sandwich Hybridization Format," Proc Natl Acad Sci USA 86(4): 1173-7 (1989), Qb Replicase and its variants (Miele et al. "Autocatalytic Replication of a Recombinant RNA." J Mol Biol 171(3): 281-95 (1983), cold-PCR (Li et al. "Replacing PCR with COLD-PCR Enriches Variant DNA Sequences and Redefines the Sensitivity of Genetic Testing." Nat Med 14(5): 579-84 (2008)) or any other nucleic acid amplification method known in the art. Depending on the amplification technique that is employed, the amplified molecules are detected during amplification (e.g., real-time PCR) or subsequent to amplification using detection techniques known to those of skill in the art. Suitable nucleic acid detection assays include, for example and without limitation, northern blot, microarray, serial analysis of gene expression (SAGE), next-generation RNA sequencing (e.g., deep sequencing, whole transcriptome sequencing, exome sequencing), gene expression analysis by massively parallel signature sequencing (MPSS), immune-derived colorimetric assays, and mass spectrometry (MS) methods (e.g., MassARRAY^{®} System).

An embodiment provided herein is a method of determining risk for a thrombotic event in a cancer patient comprising: detecting in a serum or plasma sample of a patient with cancer elevated levels of the proteins PPIA, PDIA3 and at least one of EIF5A, EIF4H, EIF4a3, UBE2N, UBE2L3, UBE2I, and HSP70 when compared with a baseline, reference, or control levels of PPIA, PDIA3 and at least one of EIF5A, EIF4H, EIF4a3, UBE2N, UBE2L3, UBE2I, and HSP70; and diagnosing the patient as at risk of a thrombotic event when PPIA, PDIA3 and at least one of EIF5A, EIF4H, EIF4a3, UBE2N, UBE2L3, UBE2I, and HSP70 are elevated over a baseline, control, or reference level.

An additional embodiment provided herein is an effective amount of isoquercetin and optionally an antithrombotic agent for use in a method of treating a thrombotic condition in a cancer patient, wherein the cancer patient has been diagnosed as being at risk of a thrombotic event by a method of determining risk of a thrombotic condition in a cancer patient, comprising the steps of a. detecting in a serum or plasma sample of a patient with cancer elevated levels of PPIA, PDIA3 and at least one of EIF5A, EIF4H, EIF4a3, UBE2N, UBE2L3, UBE2I, and HSP70 when compared with a baseline, reference, or control levels of PPIA, PDIA3 and at least one of EIF5A, EIF4H, EIF4a3, UBE2N, UBE2L3, UBE2I, and HSP70; b. diagnosing the patient as at-risk of a thrombotic condition when PPIA, PDIA3 and at least one of EIF5A, EIF4H, EIF4a3, UBE2N, UBE2L3, UBE2I, and HSP70 are elevated over a baseline, control or reference level.

A further embodiment provided herein is a method of determining risk of a thrombotic condition in a cancer patient as defined above, wherein the cancer patient is undergoing treatment with an effective amount of isoquercetin and optionally an antithrombotic agent, and wherein the method of determining risk of a thrombotic event is repeated weekly, bi-weekly, monthly, or as long as indicated throughout the course of treatment.

In certain embodiments, the patient exhibits no severe adverse events (grade 3 or 4 toxicities) during treatment.

In certain embodiments, the patient exhibits no primary venous thromboembolism (VTE) during treatment.

In certain embodiments, the patient exhibits no major hemorrhages during treatment.

Described herein and not claimed is a kit comprising a biomarker panel comprising PPIA, PDIA3 and at least one of EIF5A, EIF4H, EIF4a3, UBE2N, UBE2L3, UBE2I, and HSP70 for diagnosing a thrombotic condition in a patient in need thereof.

A kit described herein may comprise: (a) a solid support coated with polyclonal or monoclonal antibodies, wherein the antibodies comprise antibodies specific to PPIA, PDIA3 and at least one of EIF5A, EIF4H, EIF4a3, UBE2N, UBE2L3, UBE2I, and HSP70 (b) polyclonal or monoclonal antibody-substrate conjugates, wherein the substrate comprises a chromogenic or fluorescent reagent, and wherein the conjugates are reactive with the antibodies of (a); and (c) PPIA, PDIA3 and at least one of EIF5A, EIF4H, EIF4a3, UBE2N, UBE2L3, UBE2I, and HSP70 as antigen standards.

The antibodies of (a) may further comprise antibodies specific to soluble P selectin.

The solid support may be a microtiter plate or membrane. The solid support may be a bead or particle. The kit may be an ELISA kit. The solid support may be a microbead array.

A method of the claimed invention may further comprise a method of assaying PPIA, PDIA3 and at least one of EIF5A, EIF4H, EIF4a3, UBE2N, UBE2L3, UBE2I, and HSP70 in a sample of serum or plasma, the method comprising contacting the sample with the solid support and the conjugates of the kits described herein; wherein the solid support comprises a microtiter plate, wherein the conjugates comprise alkaline phosphatase, wherein the chromogenic reagent comprises p-nitrophenyl-phosphate; and assaying the reaction of the conjugates with the sample.

Further embodiments provided herein include performing an immunoassay by contacting the sample with the solid support of the kit described herein.

In certain embodiments, the immunoassay is an ELISA. In certain embodiments, the solid support is a microbead array. In certain embodiments, the sample is plasma or serum.

In certain embodiments, the method further comprises contacting the sample with the conjugates of the kit, and assaying the reaction of the conjugates with the sample.

In certain embodiments, the method further comprises contacting the antigen standards with the solid support and the conjugates, and assaying the relative levels of PPIA, PDIA3 and at least one of EIF5A, EIF4H, EIF4a3, UBE2N, UBE2L3, UBE2I, and HSP70 in the sample relative to the antigen standards.

Some embodiments provided herein include a method of determining risk for a thrombotic event in a cancer patient comprising: detecting in a serum or plasma sample of a patient with cancer elevated levels of PPIA, and PDIA3 when compared with a baseline, reference, or control levels of PPIA and PDIA3; detecting in a serum or plasma sample of the patient with cancer elevated levels of at least one of EIF5A, EIF4H, EIF4a3, UBE2N, UBE2L3, UBE2I, and HSP70 when compared with a baseline, reference, or control levels of at least one of EIF5A, EIF4H, EIF4a3, UBE2N, UBE2L3, UBE2I, and HSP70 if PPIA and PDIA3 are elevated over a baseline, control, or reference level; and diagnosing the patient as at risk of a thrombotic event when PPIA, PDIA3 and at least one of EIF5A, EIF4H, EIF4a3, UBE2N, UBE2L3, UBE2I, and HSP70 are elevated over a baseline, control, or reference level.

An additional embodiment provided herein is an effective amount of isoquercetin and optionally an antithrombotic agent for use in a method of treating a thrombotic condition in a cancer patient, wherein the cancer patient has been diagnosed as being at risk of a thrombotic event by a method comprising the steps of a. detecting in a serum or plasma sample of a patient with cancer elevated levels of PPIA, PDIA3 and at least one of EIF5A, EIF4H, EIF4a3, UBE2N, UBE2L3, UBE2I, and HSP70 when compared with a baseline, reference, or control levels of PPIA, PDIA3 and and at least one of EIF5A, EIF4H, EIF4a3, UBE2N, UBE2L3, UBE2I, and HSP70; b. diagnosing the patient as at risk of a thrombotic event when PPIA, PDIA3 and at least one of EIF5A, EIF4H, EIF4a3, UBE2N, UBE2L3, UBE2I, and HSP70 are elevated over a baseline, control, or reference level.

A further embodiment provided herein is a method for determining risk of a thrombotic event in a cancer patient according to an embodiment as described herein, wherein the method of determining risk of a thrombotic event is repeated weekly, bi-weekly, monthly, or as long as indicated throughout the course of treatment.

In certain embodiments, the patient exhibits no severe adverse events (grade 3 or 4 toxicities) during treatment.

In certain embodiments, the patient exhibits no primary venous thromboembolism (VTE) during treatment.

In certain embodiments, the patient exhibits no major hemorrhages during treatment. It is contemplated that all of the assays disclosed herein can be in kit form for use by a health care provider and/or a diagnostic laboratory.

Assays for the detection and quantitation of one or more of the UPR biomarkers can be incorporated into kits. Such kits (again kits as such are not claimed) would include probes for one or more of the UPR biomarker proteins or genes, *i.e.,* PPIA, PDIA3 and at least one of EIF5A, EIF4H, EIF4a3, UBE2N, UBE2L3, UBE2I, and HSP70, reagents for isolating and purifying the proteins or nucleic acids from biological tissue or bodily fluid, reagents for performing assays on the isolated and purified protein or nucleic acid, instructions for use, and reference values or the means for obtaining reference values in a control sample for the included protein or genes.

A kit for patient classification with regard to thrombotic risk and clinical manifestations would include probes for any combination or subset of the protein or genes from the UPR biomarker panel, i.e. PPIA, EIF5A, EIF4H, EIF4a3, UBE2N, UBE2L3, UBE2I, HSP70, and PDIA3, and optionally probes or agents for the further detection of soluble P selectin.

A kit for patient classification with regard to thrombotic risk and clinical manifestations would include probes for at least two protein or genes from the UPR biomarker panel, i.e. PPIA, PDIA3 and at least one of EIF5A, EIF4H, EIF4a3, UBE2N, UBE2L3, UBE2I, and HSP70, and optionally probes or agents for the further detection of soluble P selectin.

A kit as described herein may include reagents for testing for elevated levels of the UPR biomarker panel, i.e. PPIA, EIF5A, EIF4H, EIF4a3, UBE2N, UBE2L3, UBE2I, HSP70, and PDIA3.

A kit as described herein would include reagents for testing for elevated levels of the UPR biomarker panel, i.e. PPIA, PDIA3 and at least one of EIF5A, EIF4H, EIF4a3, UBE2N, UBE2L3, UBE2I, and HSP70.

Such a kit could include antibodies that recognize the peptide of interest, reagents for isolating and/or purifying protein from a biological tissue or bodily fluid, reagents for performing assays on the isolated and purified protein, instructions for use, and reference values or the means for obtaining reference values for the quantity or level of peptides in a control sample.

An additional kit for monitoring treatment to disease activity or progression would include probes from at least one protein or gene from the UPR biomarker panel, i.e. PPIA, EIF5A, EIF4H, EIF4a3, UBE2N, UBE2L3, UBE2I, HSP70, and PDIA3.

An additional kit for monitoring treatment to disease activity or progression would include probes from at least two proteins or genes from the UPR biomarker panel, i.e. PPIA, PDIA3 and at least one of EIF5A, EIF4a3, UBE2N, UBE2L3, UBE2I, and HSP70.

Such a kit could include antibodies that recognize the peptide of interest, reagents for isolating and/or purifying protein from a biological tissue or bodily fluid, reagents for performing assays on the isolated and purified protein, instructions for use, and reference values or the means for obtaining reference values for the quantity or level of peptides in a control sample.

These kits as described herein but not claimed would have the probes attached to a solid state. Another embodiment would have the probes in a microarray format wherein nucleic acid probes for one or more of the genes from one or more of the gene signatures would be in an ordered arrangement on a surface or substrate.

For use in the methods described herein, kits can comprise a carrier, package or container that is compartmentalized to receive one or more containers such as vials, tubes, and the like, each of the container(s) comprising one of the separate elements to be used in the method. The probes, antibodies and other reagents of the kit may be provided in any suitable form, including frozen, lyophilized, or in a pharmaceutically acceptable buffer such as TBS or PBS. The kit may also include other reagents required for utilization of the reagents in vitro or in vivo such as buffers (i.e., TBS, PBS), blocking agents (solutions including nonfat dry milk, normal sera, Tween-20 Detergent, BSA, or casein), and/or detection reagents (i.e., goat anti-mouse IgG biotin, streptavidin-HRP conjugates, allophycocyanin, B-phycoerythrin, R-phycoerythrin, peroxidase, fluors (i.e., DyLight, Cy3, Cy5, FITC, HiLyte Fluor 555, HiLyte Fluor 647), and/or staining kits (i.e., ABC Staining Kit, Pierce)). The kits may also include other reagents and/or instructions for using antibodies, probes, and other reagents in commonly utilized assays described above such as, for example, liquid or gas chromatography, spectrometry, electrochemical assay, flow cytometric analysis, ELISA, immunoblotting (i.e., western blot), immunocytochemistry, immunohistochemistry.

The kit as described herein but not claimed provides the reagent in purified form. The reagents may be immunoreagents that are provided in biotinylated form either alone or along with an avidin-conjugated detection reagent (i.e., antibody). The kit may include a fluorescently labeled immunoreagent which may be used to directly detect antigen. Buffers and the like required for using any of these systems are well-known in the art and may be prepared by the end-user or provided as a component of the kit. The kit may also include a solid support containing positive- and negative-control protein and/or tissue samples. For example, kits for performing spotting or western blot-type assays may include control cell or tissue lysates for use in SDS-PAGE or nylon or other membranes containing pre-fixed control samples with additional space for experimental samples.

The kit as described herein but not claimed will typically comprise the container described above and one or more other containers comprising materials desirable from a commercial and user standpoint, including buffers, diluents, filters, needles, syringes, and package inserts with instructions for use. In addition, a label can be provided on the container to indicate that the composition is used for a specific application, and can also indicate directions for use, such as those described above. Directions and or other information can also be included on an insert which is included with the kit.

Described herein and not claimed is a kit comprising antibodies that specifically bind the nine UPR biomarker panel PPIA, EIF5A, EIF4H, EIF4a3, UBE2N, UBE2L3, UBE2I, HSP70, and PDIA3, and, optionally, one or more additional markers such as soluble P selectin. The kit may further comprise a solid support onto which the antibodies are immobilized. Examples of a solid support include, but are not limited to, a microtiter plate, beads, a membrane or other support known to those skilled in the art. The antibodies may be immobilized via binding to antigen that is immobilized to the solid support. The antibodies may be immobilized via binding to a bead or particle such as luminex. The kit may further comprise a chromogenic substrate.

Also described herein and not claimed is a kit comprising antibodies that specifically bind the UPR biomarkers PPIA, EIF4H, PDIA3 and at least one of EIF5A, EIF4a3, UBE2N, UBE2L3, UBE2I, and HSP70, and, optionally, one or more additional markers such as soluble P selectin. The kit may further comprise a solid support onto which the antibodies are immobilized. Examples of a solid support include, but are not limited to, a microtiter plate, beads, a membrane or other support known to those skilled in the art. The antibodies may be immobilized via binding to antigen that is immobilized to the solid support. The antibodies may be immobilized via binding to a bead or particle such as luminex. The kit may further comprise a chromogenic substrate.

Also described but not claimed is an ELISA kit to screen for a plasma molecular profile in a cancer patient undergoing treatment that is predictive for thrombotic conditions such as VTE, by detecting elevated levels of PPIA, EIF5A, EIF4H, EIF4a3, UBE2N, UBE2L3, UBE2I, HSP70, and PDIA3, as disclosed herein and/or elevated levels of P selectin in plasma or serum, the kit comprising: (a) a microtiter plate coated with polyclonal or monoclonal antibodies specific to PPIA, EIF5A, EIF4H, EIF4a3, UBE2N, UBE2L3, UBE2I, HSP70, and PDIA3. disclosed herein and optionally soluble P selectin ; (b) polyclonal or monoclonal antibody-alkaline phosphatase conjugates reactive with PPIA, EIF5A, EIF4H, EIF4a3, UBE2N, UBE2L3, UBE2I, HSP70, and PDIA3 disclosed herein and optionally soluble P selectin; (c) p-nitrophenyl-phosphate; and (d) PPIA, EIF5A, EIF4H, EIF4a3, UBE2N, UBE2L3, UBE2I, HSP70, and PDIA3 as an antigen standard.

Also described herein but not claimed is an ELISA kit to screen for a plasma molecular profile in a cancer patient undergoing treatment that is predictive for thrombotic conditions such as VTE, by detecting elevated levels of PPIA, PDIA3 and at least one of EIF5A, EIF4H, EIF4a3, UBE2N, UBE2L3, UBE2I, and HSP70, as disclosed herein and/or elevated levels of P selectin in plasma or serum, the kit comprising: (a) a microtiter plate coated with polyclonal or monoclonal antibodies specific to PPIA, PDIA3 and at least one of EIF5A, EIF4H, EIF4a3, UBE2N, UBE2L3, UBE2I, and HSP70. disclosed herein and optionally soluble P selectin ; (b) polyclonal or monoclonal antibody-alkaline phosphatase conjugates reactive with PPIA, PDIA3 and at least one of EIF5A, EIF4H,EIF4a3, UBE2N, UBE2L3, UBE2I, and HSP70 disclosed herein and optionally soluble P selectin; (c) p-nitrophenyl-phosphate; and (d) PPIA, PDIA3 and at least one of EIF5A, EIF4H, EIF4a3, UBE2N, UBE2L3, UBE2I, and HSP70 as an antigen standard.

Also described herein but not claimed is an ELISA kit to screen for a plasma molecular profile in a patient that is associated with thrombotic conditions such as VTE, detecting PPIA, EIF5A, EIF4H, EIF4a3, UBE2N, UBE2L3, UBE2Im HSP70, and PDIA3 in plasma or serum, the kit comprising: (a) a microtiter plate coated with polyclonal or monoclonal antibodies specific to PPIA, EIF5A, EIF4H, EIF4a3, UBE2N, UBE2L3, UBE2I, HSP70, and PDIA3; (b) polyclonal or monoclonal antibody-alkaline phosphatase conjugates reactive with PPIA, EIF5A, EIF4H, EIF4a3, UBE2N, UBE2L3, UBE2I, HSP70, and PDIA3; (c) p-nitrophenyl-phosphate; and (d) PPIA, EIF5A, EIF4H, EIF4a3, UBE2N, UBE2L3, UBE2I, HSP70, and PDIA3 as an antigen standard.

Also described herein but not claimed is an ELISA kit to screen for a plasma molecular profile in a patient that is associated with thrombotic conditions such as VTE, detecting PPIA, PDIA3 and at least one of EIF5A, EIF4H, EIF4a3, UBE2N, UBE2L3, UBE2Im and HSP70 in plasma or serum, the kit comprising: (a) a microtiter plate coated with polyclonal or monoclonal antibodies specific to PPIA, PDIA3 and at least one of EIF5A, EIF4H, EIF4a3, UBE2N, UBE2L3, UBE2I, and HSP70; (b) polyclonal or monoclonal antibody-alkaline phosphatase conjugates reactive with PPIA, PDIA3 and at least one of EIF5A, EIF4H, EIF4a3, UBE2N, UBE2L3, UBE2I, and HSP70; (c) p-nitrophenyl-phosphate; and (d) PPIA, PDIA3 and at least one of EIF5A, EIF4H, EIF4a3, UBE2N, UBE2L3, UBE2I, and HSP70 as an antigen standard.

Also described herein but not claimed is a Luminex kit to screen in plasma, serum and\or biological fluid for a molecular profile in a patient that is associated with thrombotic conditions such as VTE, by detecting PPIA, EIF5A, EIF4H, EIF4a3, UBE2N, UBE2L3, UBE2I, HSP70, and PDIA3, and optionally P selectin, the kit comprising: (a) a microbead array coated with polyclonal or monoclonal antibodies specific to PPIA, EIF5A, EIF4H, EIF4a3, UBE2N, UBE2L3, UBE2I, HSP70, and PDIA3 and optionally P selectin; (b) polyclonal or monoclonal antibody fluorescent dye conjugates reactive with PPIA, EIF5A, EIF4H, EIF4a3, UBE2N, UBE2L3, UBE2I, HSP70, and PDIA3, and optionally P selectin (c) and PPIA, EIF5A, EIF4H, EIF4a3, UBE2N, UBE2L3, UBE2I, HSP70, and PDIA3, and optionally P selectin as an antigen standard.

Also described herein but not claimed is a Luminex kit to screen in plasma, serum and\or biological fluid for a molecular profile in a patient that is associated with thrombotic conditions such as VTE, by detecting PPIA, PDIA3 and at least one of EIF5A, EIF4H, EIF4a3, UBE2N, UBE2L3, UBE2I, and HSP70, and optionally P selectin, the kit comprising: (a) a microbead array coated with polyclonal or monoclonal antibodies specific to PPIA, PDIA3 and at least one of EIF5A, EIF4H, EIF4a3, UBE2N, UBE2L3, UBE2I, and HSP70and optionally P selectin; (b) polyclonal or monoclonal antibody fluorescent dye conjugates reactive with PPIA, PDIA3 and at least one of EIF5A, EIF4H, EIF4a3, UBE2N, UBE2L3, UBE2I, and HSP70, and optionally P selectin (c) and PPIA, PDIA3, and at least one of EIF5A, EIF4H, EIF4a3, UBE2N, UBE2L3, UBE2I, and HSP70, and, and optionally P selectin as an antigen standard.

In some embodiments, the various embodiments described herein can be combined with additional diagnostic tests including but not limited to a complete blood count (CBC), a troponin test, a CKP isoenzyme test, a comprehensive metabolic panel or any combination thereof.

### EXAMPLES

### Example 1

Tissue factor (TF) is an initiator in the coagulation cascade and is essential for hemostasis. Under pathological conditions, TF is released into the circulation on small-membrane vesicles termed microparticles (MPs). Recent studies suggest that elevated levels of MPTF may trigger thrombosis.

The unfolded protein response (UPR) is associated with malignant transformation in pancreatic cancer, but whether or not activation of the UPR is linked to cancer thrombosis has not previously been evaluated. To determine whether UPR signaling functions in the prothrombotic transformation of pancreatic cancer, pancreatic adenocarcinoma cells (HPAF-II cells) were exposed to three UPR inducers (tunicamycin, triptolide and thapsigargin) that act via independent mechanisms. Induction of UPR resulted in the release of thrombogenic material into the supernatant as evidenced by a 3-fold increase in thrombin generation in pelleted material. Release of thrombogenic material was inhibited by siRNA-mediated knockdown of UPR components including IRE1α (80% ±3% decrease) or PERK (60% ±10% decrease). Chemical inhibition of UPR also inhibited release of thrombogenic material from HPAF-II cells. The exposure to the IRE1α inhibitor, MKC-3946, resulted in a 70% ±10% decrease), and incubation with the PERK inhibitor, GSK2606414 caused an 80% ± 5% decrease in thrombin generation. Characterization of the thrombogenic activity revealed that it was present on extracellular vesicles

(EVs) and was inhibited by anti-tissue factor (anti-TF) antibodies. Flow cytometry demonstrated a 3-fold increase in the generation of TF-bearing EVs following UPR induction. Electron microscopy showed that the HPAF II EVs ranged from 100-500 µm and demonstrated increased clustering following UPR induction. Three-color immunofluorescence microscopy of HPAF II cells with labeling of actin, nuclei, and TF showed that induction of the UPR resulted in actin-poor membrane blebs rich in TF. Apoptosis as detected by caspase-3 cleavage was not observed under these conditions. Brefeldin A, which inhibits vesicular transport between the endoplasmic reticulum and the Golgi, inhibited UPR-induced generation of TF-bearing EVs, indicating that UPR-mediated vesicular trafficking contributes to TF-bearing EV formation.

To evaluate the possibility of an association between the UPR and cancer thrombosis in the clinical setting, plasmas collected from pancreatic cancer patients who were monitored prospectively for the development of venous thromboembolism were analyzed (including a lower extremity ultrasound performed at baseline and at 2 months). Proteomic analysis was performed using Somalogic technology to evaluate ~1300 analytes in plasmas from nine pancreatic cancer patients who subsequently developed venous thromboembolism and ten patients with similar pancreatic cancer characteristics who remained free of venous thromboembolism.

Plasma samples from patients with advanced pancreatic cancer were analyzed by proteomic analyses using Somascan (from SomaLogic, Inc. Boulder, Co., *See also,* Gold, L., Walker, J. J., Wilcox, S. K. & Williams, S. Advances in human proteomics at high scale with the SOMAscan proteomics platform. N. Biotechnol. 29, 543-9 (2012).) The baseline plasma was drawn (no evidence of DVT with baseline ultrasound) and the patient continued to be monitored for development of VTE for 8 weeks (See MicroTec Trial as described in Zwicker et al., Br. J. Haematol. 2013 Feb;160(4):530-7). A nine protein UPR panel based on a commercially available UPR gene list was evaluated. As shown in Fig. 1, a score (0 or 1) based on above or below the median concentration for each protein, was determined. Table 1 below shows p-values for individual UPR markers.

**Table 1 - Individual UPR marker p-values**

| Protein | p-value |
|---|---|
| PPIA | 0.0002 |
| EIF5A | 1.0 |
| EIF4H | 0.009 |
| EIF4A3 | 0.10 |
| UBE2N | 0.48 |
| UBE2L3 | 1.0 |
| UBE2I | 0.10 |
| HSP70 | 0.26 |
| PDIA3 | 0.009 |

As shown in **Fig. 1****,** evaluation of nine UPR markers present in the SOMA-scan panel demonstrated significant upregulation in plasmas of patients who developed clots compared to those who did not (p = 0.0001), see in particular the elevated levels of PPIA, EIF5A, EIF4H, EIF4a3, UBE2N, UBE2L3, UBE2I, HSP70, and PDIA3. These data support a model whereby activation of the UPR results in increased vesicular trafficking leading to the release of TF-bearing EVs. These observations indicate a mechanistic link between tumor progression in pancreatic cancer and cancer-associated thrombosis. It is likely that similar results will be found in additional cancer patients, including those without advanced disease. Additionally, it will be beneficial to continue to monitor patients for elevated levels of the UPR markers every 2 weeks, or monthly, or as long as they are being treated or are at risk for thrombotic conditions. Blood is drawn by peripheral venipuncture into 3.2% citrate. Plasma is separated at 2100g for 20 minutes within one hour of specimen collection. A second centrifugation is performed at 2100g for 20 minutes to generate platelet-free plasma and stored in aliquots at -80°C until analysis.

The primary VTE endpoint include any symptomatic proximal or distal deep vein thrombosis, symptomatic PE or fatal PE diagnosed by autopsy, asymptomatic proximal DVT diagnosed by protocol-specified ultrasound at end of study. All suspected VTE are assessed by an independent adjudication committee that included central radiologic review of images. Criteria for new VTE include any of the following: A) A new noncompressibility of lower extremity deep venous segments by compression ultrasound (distal lower extremity thrombus qualified for primary VTE endpoint only if symptomatic). B) Intraluminal defects in two or more views on pulmonary angiography, sudden contrast cut-off of one or more vessels greater than 2.5 mm in diameter on a pulmonary angiogram; a high probability VQ lung scan showing one or more segmental perfusion defects with corresponding normal ventilation (mismatch defect); or abnormal spiral CT showing thrombus in pulmonary vessels (subsegmental or larger). All other venous or arterial events are recorded and analyzed as secondary endpoints. Criteria for major hemorrhage was according to ISTH definition (Schulman S, and Kearon C. J Thromb Haemost. 2005;3(4):692-4). All toxicities were graded according NCI Common Terminology Criteria for Adverse Events (CTCAE). Study oversight was performed by an independent Data Safety Monitoring Committee at Dana Farber Harvard Cancer Center.

### GENERAL METHODS

Standard methods in molecular biology are described Sambrook, Fritsch and Maniatis (1982 & 1989 2nd Edition, 2001 3rd Edition) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA). Standard methods also appear in Ausbel, et al. (2001) Current Protocols in Molecular Biology, Vols.1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, centrifugation, and crystallization are described (Coligan, et al. (2000) Current Protocols in Protein Science, Vol. 1, John Wiley and Sons, Inc., New York). Chemical analysis, chemical modification, post-translational modification, production of fusion proteins, glycosylation of proteins are described (see, *e.g.,* Coligan, et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391). Production, purification, and fragmentation of polyclonal and monoclonal antibodies are described (Coligan, et al. (2001) Current Protcols in Immunology, Vol. 1, John Wiley and Sons, Inc., New York; Harlow and Lane (1999) Using Antibodies, Cold Spring Harbor Laboratory Press, Cold Spring Harbor, NY; Harlow and Lane, *supra*)*.* Standard techniques for characterizing ligand/receptor interactions are available (see, *e.g.,* Coligan, et al. (2001) Current Protocols in Immunology, Vol. 4, John Wiley, Inc., New York).

Various modifications of the invention in addition to those described herein will become apparent to those skilled in the art from the foregoing description and the accompanying figures. Such modifications are intended to fall within the scope of the appended claims.

The foregoing written specification is considered to be sufficient to enable one skilled in the art to practice the invention. Various modifications of the invention in addition to those shown and described herein will become apparent to those skilled in the art from the foregoing description and fall within the scope of the appended claims.

## Claims

1. A method of determining risk for a thrombotic event in a cancer patient comprising:
detecting in a serum or plasma sample obtained from a patient with cancer elevated levels of the proteins Peptidyl-prolyl cis-trans isomerase A (PPIA), Protein disulfide-isomerase A3 (PDIA3) and at least one of Eukaryotic translation initiation factor 5A-1 (EIF5A), Eukaryotic translation initiation factor 4H (EIF4H), Eukaryotic initiation factor 4A-III (EIF4a3), Ubiquitin-conjugating enzyme E2 N (UBE2N), Ubiquitin-conjugating enzyme E2 L3 (UBE2L3), SUMO-conjugating enzyme UBC9 (UBE2I), and Heat shock 70 kDa protein 1A (HSP70), when compared with a baseline, reference, or control levels of PPIA, PDIA3 and at least one of EIF5A, EIF4H, EIF4a3, UBE2N, UBE2L3, UBE2I, and HSP70; and diagnosing the patient as at risk of a thrombotic event when PPIA, PDIA3 and at least one of EIF5A, EIF4H, EIF4a3, UBE2N, UBE2L3, UBE2I, and HSP70 are elevated over a baseline, control, or reference level.

2. An effective amount of isoquercetin and optionally an antithrombotic agent for use in a method of treating a thrombotic condition in a cancer patient, wherein the cancer patient has been diagnosed as being at risk of a thrombotic event by a method according to claim 1.

3. A method according to claim 1, wherein the patient is undergoing treatment with an effective amount of isoquercetin and optionally an antithrombotic agent; wherein the method of determining risk for a thrombotic event is repeated weekly, bi-weekly, monthly, or as long as indicated throughout the course of treatment.

4. The method of claim 1 or 3, wherein the patient exhibits i) no severe adverse events during treatment; ii) no primary venous thromboembolism (VTE) during treatment; and/or iii) no major hemorrhages during treatment.

5. The method of claim 4 wherein a severe adverse event is a grade 3 or 4 toxicity.

6. A method according to any one of claims 1, or 3 to 5 wherein the method comprises i) contacting the serum or plasma sample with (a) a solid support coated with polyclonal or monoclonal antibodies, wherein the antibodies comprise antibodies specific to PPIA, PDIA3 and at least one of EIF5A, EIF4H, EIF4a3, UBE2N, UBE2L3, UBE2I, and HSP70; and (b) polyclonal or monoclonal antibody-substrate conjugates, wherein the substrate comprises a chromogenic or fluorescent reagent, and wherein the conjugates are reactive with the antibodies of (a); and (c) PPIA, PDIA3 and at least one of EIF5A, EIF4H, EIF4a3, UBE2N, UBE2L3, UBE2I, and HSP70, as antigen standards.

7. A method according to claim 6 wherein the solid support comprises a microtiter plate, wherein the conjugates comprise alkaline phosphatase, wherein the chromogenic reagent comprises p-nitrophenyl-phosphate; and ii) assaying the reaction of the conjugates with the sample.

8. The method of claim 6 or 7, wherein the assay is an ELISA.

9. The method of any one of claims 6 to 8, wherein the solid support is a microbead array.

10. The method of any one of claims 6 to 9, further comprising contacting the antigen standards with the solid support and the conjugates, and assaying the relative levels of PPIA, PDIA3 and at least one of EIF5A, EIF4H, EIF4a3, UBE2N, UBE2L3, UBE2I, and HSP70 in the sample relative to the antigen standards.

11. The method of any one of claims 1 and 3 to 10 wherein the patient has advanced cancer.

12. An effective amount of isoquercetin and optionally an antithrombotic agent for use in a method of treating a thrombotic condition in a cancer patient according to claim 2, wherein the patient has advanced cancer.

## Patentansprüche

1. Verfahren zur Bestimmung des Risikos eines thrombotischen Ereignisses bei einem Krebspatienten, umfassend:
Nachweisen, in einer Serum- oder Plasmaprobe, die aus einem Patienten mit Krebs erhalten wurde, von erhöhten Spiegeln der Proteine Peptidyl-Prolyl-cis-trans-Isomerase A (PPIA), Protein-Disulfid-Isomerase A3 (PDIA3) und mindestens einem von eukaryotischem Translationinitiationsfaktor 5A-1 (EIF5A), eukaryotischem Translationinitiationsfaktor 4H (EIF4H), eukaryotischem Initiationsfaktor 4A-III (EIF4a3), Ubiquitin-konjugierendem Enzym E2 N (UBE2N), Ubiquitin-konjugierendem Enzym E2 L3 (UBE2L3), SUMO-konjugierendem Enzym UBC9 (UBE2I) und Hitzeschock-70-kDa-Protein 1A (HSP70), beim Vergleich mit Baseline-, Referenz- oder Kontrollniveaus von PPIA, PDIA3 und mindestens einem von EIF5A, EIF4H, EIF4a3, UBE2N, UBE2L3, UBE2I und HSP70; und Diagnostizieren, dass bei dem Patienten das Risiko eines thrombotischen Ereignisses besteht, wenn PPIA, PDIA3 und mindestens eines von EIF5A, EIF4H, EIF4a3, UBE2N, UBE2L3, UBE2I und HSP70 über ein Baseline-, Kontroll- oder Referenzniveau erhöht sind.

2. Wirksame Menge an Isoquercetin und optional einem antithrombotischen Mittel zur Verwendung in einem Verfahren zur Behandlung einer Thrombose bei einem Krebspatienten, wobei diagnostiziert wurde, dass bei dem Krebspatienten ein Risiko eines thrombotischen Ereignisses besteht, durch ein Verfahren nach Anspruch 1.

3. Verfahren nach Anspruch 1, wobei sich der Patient einer Behandlung mit einer wirksamen Menge an Isoquercetin und optional einem antithrombotischen Mittel unterzieht; wobei das Verfahren zur Bestimmung des Risikos eines thrombotischen Ereignisses wöchentlich, zweiwöchentlich, monatlich oder so lange wie angezeigt während des gesamten Behandlungsverlaufs wiederholt wird.

4. Verfahren nach Anspruch 1 oder 3, wobei der Patient i) keine schweren unerwünschten Ereignisse während der Behandlung; ii) keine primäre venöse Thromboembolie (VTE) während der Behandlung; und/oder iii) keine schwerwiegenden Blutungen während der Behandlung aufweist.

5. Verfahren nach Anspruch 4, wobei ein schweres unerwünschtes Ereignis eine Toxizität vom Grad 3 oder 4 ist.

6. Verfahren nach einem der Ansprüche 1 oder 3 bis 5, wobei das Verfahren umfasst i) Inkontaktbringen der Serum- oder Plasmaprobe mit (a) einem festen Träger, der mit polyklonalen oder monoklonalen Antikörpern beschichtet ist, wobei die Antikörper Antikörper umfassen, die für PPIA, PDIA3 und mindestens eines von EIF5A, EIF4H, EIF4a3, UBE2N, UBE2L3, UBE2I und HSP70 spezifisch sind; und (b) polyklonalen oder monoklonalen Antikörper-Substrat-Konjugaten, wobei das Substrat ein chromogenes oder fluoreszierendes Reagens umfasst und wobei die Konjugate reaktiv sind mit den Antikörpern von (a); und (c) PPIA, PDIA3 und mindestens einem von EIF5A, EIF4H, EIF4a3, UBE2N, UBE2L3, UBE2I und HSP70 als Antigenstandards.

7. Verfahren nach Anspruch 6, wobei der feste Träger eine Mikrotiterplatte umfasst, wobei die Konjugate alkalische Phosphatase umfassen, wobei das chromogene Reagens p-Nitrophenylphosphat umfasst; und ii) Testen der Reaktion der Konjugate mit der Probe.

8. Verfahren nach Anspruch 6 oder 7, wobei der Assay ein ELISA ist.

9. Verfahren nach einem der Ansprüche 6 bis 8, wobei der feste Träger ein Mikrokügelchen-Array ist.

10. Verfahren nach einem der Ansprüche 6 bis 9, ferner umfassend das Inkontaktbringen der Antigenstandards mit dem festen Träger und den Konjugaten und Testen der relativen Niveaus von PPIA, PDIA3 und mindestens einem von EIF5A, EIF4H, EIF4a3, UBE2N, UBE2L3, UBE2I und HSP70 in der Probe relativ zu den Antigenstandards.

11. Verfahren nach einem der Ansprüche 1 und 3 bis 10, wobei der Patient fortgeschrittenen Krebs hat.

12. Wirksame Menge an Isoquercetin und optional einem antithrombotischen Mittel zur Verwendung in einem Verfahren zur Behandlung einer Thrombose bei einem Krebspatienten nach Anspruch 2, wobei der Patient fortgeschrittenen Krebs hat.

## Revendications

1. Méthode de détermination du risque d'un événement thrombotique chez un patient atteint d'un cancer comprenant :
la détection dans un échantillon de sérum ou de plasma prélevé chez un patient atteint d'un cancer de taux élevés des protéines peptidyl-prolyl cis-trans isomérase A (PPIA), protéine disulfure-isomérase A3 (PDIA3) et d'au moins un parmi le facteur d'initiation de traduction eucaryote 5A-1 (EIF5A), le facteur d'initiation de traduction eucaryote 4H (EIF4H), le facteur d'initiation eucaryote 4A-III (EIF4a3), l'enzyme de conjugaison d'ubiquitine E2 N (UBE2N), l'enzyme de conjugaison d'ubiquitine E2 L3 (UBE2L3), l'enzyme de conjugaison de SUMO UBC9 (UBE2I) et la protéine de choc thermique de 70 kDa 1A (HSP70), lorsqu'ils sont comparés à un taux de ligne de base, de référence ou témoin de PPIA, de PDIA3 et d'au moins un parmi EIF5A, EIF4H, EIF4a3, UBE2N, UBE2L3, UBE21 et HSP70 ; et
le diagnostic du patient comme présentant le risque d'un événement thrombotique lorsque la PPIA, la PDIA3 et au moins un parmi EIF5A, EIF4H, EIF4a3, UBE2N, UBE2L3, UBE2I, et HSP70 sont élevés par rapport à un taux de ligne de base, de référence ou témoin.

2. Quantité efficace d'isoquercétine et éventuellement d'un agent antithrombotique destinée à être utilisée dans une méthode de traitement d'un état thrombotique chez un patient atteint d'un cancer, ledit patient atteint d'un cancer ayant été diagnostiqué comme présentant le risque d'un événement thrombotique par une méthode selon la revendication 1.

3. Méthode selon la revendication 1, ledit patient suivant un traitement avec une quantité efficace d'isoquercétine et éventuellement un agent antithrombotique ; ladite méthode de détermination du risque d'un événement thrombotique étant répété toutes les semaines, toutes les deux semaines, tous les mois ou aussi longtemps que cela est indiqué tout au long du traitement.

4. Méthode selon la revendication 1 ou 3, ledit patient ne présentant i) aucun événement indésirable grave pendant le traitement ; ii) aucune thrombo-embolie veineuse (TEV) primaire pendant le traitement ; et/ou iii) aucune hémorragie majeure pendant le traitement.

5. Méthode selon la revendication 4, un événement indésirable grave étant une toxicité de grade 3 ou 4.

6. Méthode selon l'une quelconque des revendications 1 ou 3 à 5, ladite méthode comprenant i) la mise en contact de l'échantillon de sérum ou de plasma avec
(a) un support solide revêtu d'anticorps polyclonaux ou monoclonaux, lesdits anticorps comprenant des anticorps spécifiques à PPIA, à PDIA3 et à au moins un parmi EIF5A, EIF4H, EIF4a3, UBE2N, UBE2L3, UBE21 et HSP70 ; et (b) des conjugués anticorps-substrat polyclonaux ou monoclonaux, le substrat comprenant un réactif chromogène ou fluorescent, et lesdits conjugués étant réactifs avec les anticorps de (a) ; et (c) de la PPIA, de la PDIA3 et au moins un parmi EIF5A, EIF4H, EIF4a3, UBE2N, UBE2L3, UBE21 et HSP70, en tant qu'étalons d'antigènes.

7. Méthode selon la revendication 6, ledit support solide comprenant une plaque de microtitrage, lesdits conjugués comprenant de la phosphatase alcaline, ledit réactif chromogène comprenant du p-nitrophényl-phosphate ; et ii) le dosage de la réaction des conjugués avec l'échantillon.

8. Méthode selon la revendication 6 ou 7, ledit dosage étant un dosage ELISA.

9. Méthode selon l'une quelconque des revendications 6 à 8, ledit support solide étant un réseau de microbilles.

10. Méthode selon l'une quelconque des revendications 6 à 9, comprenant en outre la mise en contact des étalons d'antigènes avec le support solide et les conjugués, et le dosage des taux relatifs de PPIA, de PDIA3 et d'au moins un parmi EIF5A, EIF4H, EIF4a3, UBE2N, UBE2L3, UBE21 et HSP70 dans l'échantillon par rapport aux étalons d'antigènes.

11. Méthode selon l'une quelconque des revendications 1 et 3 à 10, ledit patient étant atteint d'un cancer avancé.

12. Quantité efficace d'isoquercétine et éventuellement d'un agent antithrombotique destinée à être utilisée dans une méthode de traitement d'un état thrombotique chez un patient atteint d'un cancer selon la revendication 2, ledit patient étant atteint d'un cancer avancé.
